(19) Europäisches Patentamt
European Patent Office
Office européen des brevets

(11) Publication number: 0 120 613 A1

# (12) EUROPEAN PATENT APPLICATION

(21) Application number: 84301255.0

(22) Date of filing: 27.02.84

(51) Int. Cl.³: C 07 D 499/00
C 07 D 405/06, C 07 D 409/06
A 61 K 31/43
//C07D407/04, C07D307/68,
C07F7/10, C07F7/18, C07F9/65

(30) Priority: 02.03.83 GB 8305704
16.11.83 GB 8330516

(43) Date of publication of application:
03.10.84 Bulletin 84/40

(84) Designated Contracting States:
BE CH DE FR GB IT LI NL SE

(71) Applicant: BEECHAM GROUP PLC
Beecham House Great West Road
Brentford Middlesex(GB)

(72) Inventor: Osborne, Neal Frederick
9 Steeres Hill
Rusper West Sussex(GB)

(74) Representative: Hardman, Carol Pauline et al,
Beechams Pharmaceuticals Great Burgh Yew Tree
Bottom Road
Epsom, Surrey, KT18 5XQ(GB)

(54) Penem derivatives and precursors.

(57) A compound of formula (II):

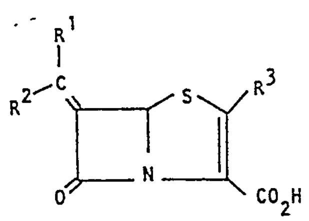

(II)

or a pharmaceutically acceptable salt or *in vivo* hydrolysable ester thereof, wherein one of $R^1$ or $R^2$ is hydrogen, and the other is a group of subformula (a):

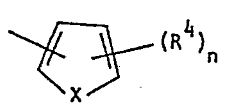

(a)

wherein $R^4$ is a substituent group, X is an oxygen atom, a sulphur atom or an $-NR^5$ group wherein $R^5$ is hydrogen hydrocarbon or a nitrogen protecting group, and n is 0, 1, 2 or 3; and $R^3$ represents hydrogen or an organic group.

Processes for preparing these compounds and pharmaceutical compositions containing them are also described.



Croydon Printing Company Ltd.

NOVEL COMPOUNDS

This invention relates to β-lactam compounds and in particular to a class of 6-alkylidene penems which have antibacterial and β-lactamase inhibitory properties. The compounds are therefore useful in the treatment of antibacterial infections in humans or animals, either alone or in combination with other antibiotics.

European Patent Publication No 0041 768 (corresponding to US Serial No 257 481) discloses 6-alkylidene-2-penems of the general formula (I):

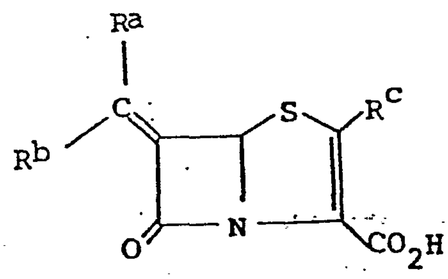

(I)

wherein $R^a$, and $R^b$ represent hydrogen or an optionally substituted hydrocarbon or heterocyclic group, and $R^c$ represents hydrogen or an organic group, which possess antibacterial activity. The compounds also inhibit β-lactamases and have a synergistic effect in combination with other β-lactam antibiotics.

The Applicants have found that certain of these compounds have better activity than others within the class

According to the present invention there is provided a compound of formula (II):

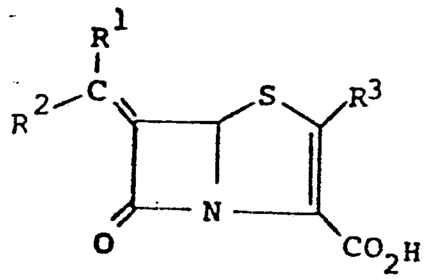

(II)

or a pharmaceutically acceptable salt or in vivo hydrolysable ester thereof, wherein one of $R^1$ or $R^2$ is hydrogen, and the other is a group of subformula (a):

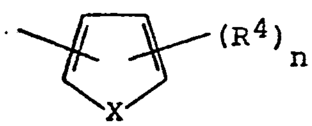

(a)

wherein $R^4$ is a substituent group, X is an oxygen atom, a sulphur atom or an $-NR^5$ group wherein $R^5$ is hydrogen, hydrocarbon or a nitrogen protecting group, and n is 0, 1, 2 or 3;
and $R^3$ represents hydrogen or an organic group.

Suitable substituent groups $R^4$ include $(C_{1-6})$alkanoyl, $(C_{1-6})$ alkanoyloxy in particular acetoxy, heterocyclyl, amino, $C_{1-6}$ alkanoyl-amino, mono, di- and tri- $(C_{1-6})$ alkylamino, hydroxy, $C_{1-6}$ alkoxy, sulpho, mercapto, $C_{1-6}$ alkylthio, $C_{1-6}$ alkylsulphinyl, $C_{1-6}$ alkyl sulphonyl, heterocyclyl-thio, arylthio, sulphamoyl, carbamoyl, amidino, guanidino, nitro, halogen, carboxy and salts and esters thereof, arylcarbonyl or heterocyclylcarbonyl, or optionally substituted $(C_{1-6})$ alkyl, $(C_{2-6})$alkenyl, $(C_{2-6})$ alkynyl, aryl, and aryl

($C_{1-6}$) alkyl wherein the optional substituent(s) are those mentioned above for $R^4$ itself.

When $R^4$ is a salt or ester of carboxy, it is suitably a pharmaceutically acceptable salt such as the sodium salt or pharmaceutically acceptable ester such as the methyl ester.

Suitable substituent groups $R^4$ include acetoxymethyl, bromo, sodium salt of carboxy or methyl ester of carboxy.

Preferably n is zero.

The term 'heterocyclyl' includes single or fused rings comprising up to four hetero atoms in the ring selected from oxygen, nitrogen and sulphur and optionally substituted with up to three halogen, $C_{1-6}$ alkyl, $C_{1-6}$ alkoxy, halo-($C_{1-6}$)-alkyl, hydroxy, amino, carboxy, $C_{1-6}$ alkoxycarbonyl, $C_{1-6}$ alkoxycarbonyl($C_{1-6}$) alkyl, aryl or oxo groups.

When used herein the term 'aryl' includes phenyl and naphthyl optionally substituted with up to five, preferably up to three, groups selected from halogen, $C_{1-6}$ alkyl, phenyl, $C_{1-6}$ alkoxy, halo($C_{1-6}$) alkyl, hydroxy, amino, nitro, carboxy, $C_{1-6}$ alkoxycarbonyl, $C_{1-6}$ alkoxycarbonyl-($C_{1-6}$)-alkyl, $C_{1-6}$ alkylcarbonyloxy, or $C_{1-6}$ alkylcarbonyl groups.

The term 'hydrocarbon' includes groups having up to 18 carbon atoms, suitably up to 10 carbon atoms, conveniently up to 6 carbon atoms. Suitable hydrocarbon groups include $C_{1-6}$ alkyl, $C_{2-6}$ alkenyl, $C_{2-6}$ alkynyl, $C_{3-7}$ cycloalkyl, $C_{3-7}$ cycloalkyl($C_{1-6}$)-alkyl, aryl, and aryl($C_{1-6}$)alkyl.

Preferably $R^1$ or $R^2$ is a 2-furyl, 3-furyl, 2-thienyl or 3-thienyl group.

When $R^5$ is a nitrogen protecting group it is suitably one which is well known in the art such as carboxyethyl, p-nitrobenzyloxycarbonyl, benzyloxycarbonyl or $SO_2R^q$ wherein $R^q$ is a hydrocarbon group.

Suitably $R^5$ is hydrogen or a $C_{1-6}$ alkyl group in particular methyl.

Suitably $R^3$ represents hydrogen or an organic group linked through a sulphur or carbon atom. For example $R^3$ may represent hydrogen or a group of formula $R^a$ or $SR^a$ where $R^a$ is an optionally substituted $C_{1-10}$ hydrocarbon or heterocyclic group. Preferably, $R^3$ represents hydrogen, optionally substituted $C_{1-10}$ alkyl or optionally substituted $C_{1-10}$ alkylthio, wherein the substituent is hydroxy, $C_{1-6}$ alkoxy, $C_{1-6}$ alkanoyloxy, halogen, mercapto, $C_{1-6}$ alkylthio, heterocyclylthio, amino, mono- or di($C_{1-6}$)alkylamino, $C_{1-6}$ alkanoylamino, carboxy, or $C_{1-6}$ alkoxycarbonyl.

Pharmaceutically acceptable in vivo hydrolysable esters are those esters which hydrolyse in the human body to produce the parent acid or its salt. Such esters may be identified by administration to a test animal such as a rat or a mouse by intravenous administration and thereafter examining the test animal's body fluids for the presence of the compound of the formula (II) or its salt.

Suitable esters groups of this type include those of part formulae (b), (c) and (d):

$$\diagdown CO_2\underset{|}{\overset{A^1}{C}}H\text{-}O.CO.A^2 \qquad (b)$$

$$\diagdown CO_2\text{-}A^3\text{-}N(A^4)(A^5) \qquad (c)$$

$$\diagdown CO_2CH_2\text{-}OA^6 \qquad (d)$$

wherein $A^1$ is hydrogen, methyl, or phenyl, $A^2$ is $C_{1-6}$ alkyl, $C_{1-6}$ alkoxy or phenyl; or $A^1$ and $A^2$ together

or two methoxy groups; $A^3$ represents $C_{1-6}$ alkylene optionally substituted with a methyl or ethyl group $A^4$ and $A^5$ independently represent $C_{1-6}$ alkyl; $A^6$ represents $C_{1-6}$ alkyl. Examples of suitable in vivo hydrolysable ester groups include acetoxymethyl, pivaloyloxymethyl, α-acetoxyethyl, α-acetoxybenzyl, α-pivaloyloxyethyl, ethoxycarbonyloxymethyl, α-ethoxycarbonyloxyethyl, dimethylaminomethyl, diethylaminomethyl, phthalidyl and dimethoxy-phthalidyl groups.

Suitable pharmaceutically acceptable salts of the 3-carboxylic acid group of the compound of formula (II) include metal salts, eg aluminium, alkali metal salts such as sodium or potassium, alkaline earth metal salts such as calcium or magnesium, and ammonium or substituted ammonium salts for example those with lower alkylamines such triethylamine, hydroxy-lower alkylamines such as 2-hydroxyethylamine, bis-(2-hydroxyethyl)-amine or

tri-(2-hydroxyethyl)-amine, cycloalkylamines such as bicyclohexylamine, or with procaine, dibenzylamine, N,N-dibenzylethylenediamine, 1-ephenamine, N-ethylpiperadine, N-benzyl-β-phenethylamine, dehydroabietylamine, N,N'-bishydroabietylethylenediamine, or bases of the pyridine type such as pyridine, collidine or quinoline, or other amines which have been used to form salts with known penicillins.

Suitable groups for $R^3$ include hydrogen, or methyl, ethyl, propyl, methylthio, ethylthio, methylsulphinyl, ethylsulphinyl, hydroxymethyl, methoxymethyl, ethoxymethyl, acetoxymethyl, 1-, or 2-acetoxyethyl, aminomethyl, 2-aminoethyl, acetamidomethyl, 2-acetamidoethyl, carboxymethyl, hydroxymethylthio, 2-hydroxyethylthio, methoxymethylthio, 2-methoxyethylthio, acetoxymethylthio, aminomethylthio, 2-aminoethylthio, acetamidomethylthio, 2-acetamidoethylthio, carboxymethylthio, 2-carboxyethylthio or pyridylthio.

Particular groups for $R^3$ are hydrogen aminomethyl, aminoethyl, methylthio, ethylthio, 2-aminoethylthio, 2-hydroxyethylthio, 2-carboxyethylthio, methyl, ethyl, acetamidoethyl, pyridylthio, and ethylsulphinyl.

The compounds of formula (II) may exist in two optically active forms and it is to be understood that both such forms as well as racemic mixtures thereof are embraced by the present invention. It is believed that the more active form is that of structure (IIa):

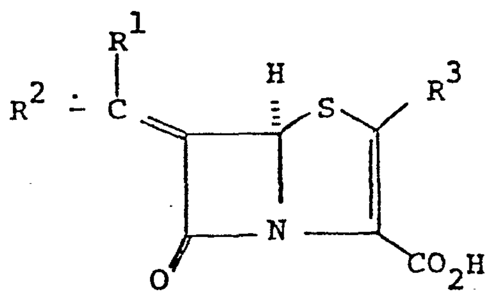

(IIa)

wherein $R^1$ is the group of sub-formula (a) as hereinbefore defined.

Suitable compounds of formula (II) include:

(5 RS) p-Nitrobenzyl (Z)-6-Furfurylidenepenem-3-carboxylate;

(5 RS) Sodium (Z)-6-Furfurylidenepenem-3-carboxylate;

(5 RS) p-Nitrobenzyl 2-Ethylthio-6-furfurylidenepenem-3-carboxylate;

(5 RS) Sodium 2-Ethylthio-(Z)-6-furfurylidenepenem-3-carboxylate;

(5 RS) p-Nitrobenzyl (Z)-6-Furfurylidene-2-(2-hydroxyethylthio) penem-3-carboxylate;

(5 RS) Sodium (Z)-6-Furfurylidene-2-(2-hydroxy ethylthio) penem-3-carboxylate;

(5 RS) p-Nitrobenzyl (Z)-6- (5-Acetoxymethylfur furylidene) penem-3-carboxylate;

(5 RS) Sodium (Z)-6- (5-Acetoxymethylfurfurylidene) penem-3-carboxylate;

(5 RS) p-Nitrobenzyl 6-(5-Acetoxymethylfurfur ylidene)-2-ethylthiopenem-3-carboxylate;

(5 RS) Sodium (Z)-6-(5-Acetoxymethylfurfurylidene) -2-ethylthiopenem-3-carboxylate;

(5 RS) p-Nitrobenzyl (Z)-6- 5-[(Z)-2-methoxy

carbonylvinylfurfurylidene] penem-3-carboxylate;

(5 RS) Sodium (Z)-6- 5-[(Z)-2-methoxycarbonyl-vinylfurfurylidene] penem-3-carboxylate;

(5 RS) p-Nitrobenzyl 6-(2-thienyl)methylenepenem-3-carboxylate;

(5 RS) Sodium (Z)-6-(2-thienyl)methylenepenem-3-carboxylate;

(5 RS) Sodium (E)-6-(2-thienyl)methylenepenem-3-carboxylate;

(5 RS) p-Nitrobenzyl 6-(3-thienyl)methylenepenem-3-carboxylate;

(5 RS) Sodium (Z)-6-(3-thienyl)methylenepenem-3-carboxylate;

(5 RS) Sodium (E)-6-(3-thienyl)methylenepenem-3-carboxylate;

(5 RS) p-Nitrobenzyl (E)-6-furfurylidenepenem-3-carboxylate;

(5 RS) p-Nitrobenzyl (Z)-6-furfurylidenepenem-3-carboxylate;

(5 RS) Sodium (E)-6-furfurylidenepenem-3-carboxylate;

(5 RS) 2-(2-Carboxyethylthio)-(Z)-6-furfurylidene-penem-3-carboxylate, Disodium salt;

(5 RS) p-Nitrobenzyl (Z)-6-(5-Bromofurfurylidene) penem-3-carboxylate;

(5 RS) Sodium (Z)-6-(5-Bromofurfurylidene)penem-3-carboxylate;

(5 RS) p-Nitrobenzyl (E)-6-(3-Furylmethylene)penem-3-carboxylate;

(5 RS) p-Nitrobenzyl (Z)-6-(3-Furylmethylene)penem-3-carboxylate;

(5 RS) Sodium (E)-6-(3-Furylmethylene)penem-3-carboxylate;

(5 RS) Sodium (Z)-6-(3-Furylmethylene)penem-3-carboxylate;

(5 RS) p-Nitrobenzyl (Z)-6-(5-p-Nitrobenzyloxycarbonyl-furfurylidene)penem-3-carboxylate; and

(5 RS) (Z)-6-(5-Carboxyfurfurylidene)penem-3-carboxylate, Disodium salt.

Further according to the present invention there is provided a process for preparing a compound of formula II as defined in relation to formula II or a pharmaceutically acceptble salt or in-vivo hydrolysable ester thereof which process comprises the elimination of the elements H-Z from a compound of formula (III)

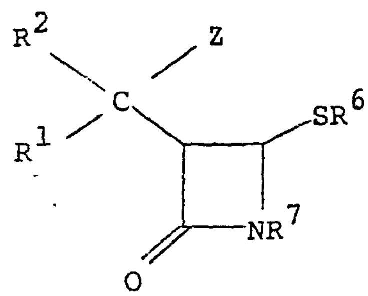

(III)

wherein $R^1$ and $R^2$ are as defined in relation to formula II; Z is hydroxy or a leaving group;
$R^6$ is $C_{1-6}$ alkyl, aryl or aryl ($C_{1-6}$) alkyl;
$R^7$ is hydrogen or an N-protecting group; or $R^6$ and $R^7$ are joined together to form a penem ring such that the compound is of formula (IV)

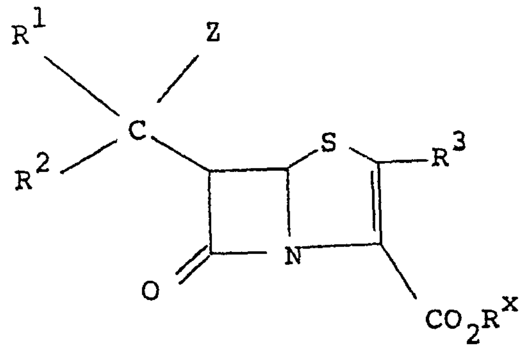

(IV)

wherein $R^1$, $R^2$ and $R^3$ are as defined in relation to formula (II),

Z is as defined above; and

$R^x$ is a hydrogen or carboxy blocking group; and thereafter if necessary carrying out one or more of the following steps:

i) joining $R^6$ and $R^7$ to complete the penem ring of formula (II);

ii) removing the carboxy blocking group $R^x$; and

iii) converting the product into a pharmaceutically acceptable salt or <u>in-vivo</u> hydrolysable ester.

The compounds of this invention may be prepared by dehydration of a compound of formula (V):

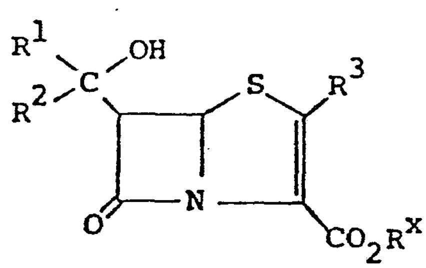

(V)

wherein $R^1$, $R^2$ and $R^3$ are as defined with respect to formula (II) above and $R^x$ represents hydrogen, or a carboxyl-blocking group, and thereafter if necessary carrying out one or more of the following steps:

i) removal of any carboxyl blocking group $R^x$;

ii) converting the product to a pharmaceutically acceptable salt or in vivo hydrolysable ester group.

Suitable carboxyl-blocking derivatives for the group $-CO_2R^x$ in formulae (IV) and (V) include salts, ester, and anhydride derivatives of the carboxylic acid. The derivative is one which may readily be cleaved at a later stage of the reaction. The salts may not be pharmaceutically acceptable. Suitable salts include inorganic salts, for example metal salts such silver or mercuric salt, or alkali metal salts such as the lithium or sodium salt, tertiary amine salts, such as thos with tri-lower-alkylamines, N-ethylpiperadine, dimethylpiperazine. A preferred salt is with triethylamine.

Suitable ester-forming carboxyl-protecting groups are those which may be removed under conventional conditions. Such groups for $R^x$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, allyl, acetonyl, t-butyl, t-amyl, diphenylmethyl, triphenylmethyl, adamantyl, 2-benzyl-oxyphenyl, 4-methylthiophenyl, tetrahydrofur-2-yl, tetrahydropyran-2-yl, pentachlorophenyl, p-toluene-sulphonylethyl, methoxymethyl, a silyl, stannyl or phosphorus-containing group, an oxime radical of formula $-N=CHR^o$ where $R^o$ is aryl or heterocyclic, or an in vivo hydrolysable ester radical such as defined above.

The carboxyl group may be regenerated from any of the above esters by usual methods appropriate to the particular $R^x$ group, for example, acid - and base - catalysed hydrolysis, or by enzymically - catalysed hydrolysis, or by hydrogenation. The hydrolysis must

of course be carried out under conditions to which the groups on the rest of the molecule are stable.

When it is desired to produce a compound of formula (II) in the form of a free acid or salt by the process of this invention, a compound of formula (V) is generally employed wherein $R^x$ is a carboxyl-blocking group. For the preparation of a compound of formula (II) in the form of a pharmaceutically acceptable ester, it is convenient to employ a compound of formula (V) wherein $R^x$ represents the desired ester group.

The dehydration process of this invention may be carried out by treating a compound of formula (V) with a compound of formula (VI):

$$R^8O_2C-N=N-CO_2R^9 \qquad (VI)$$

wherein $R^8$ and $R^9$ are independently aryl, or $C_{1-6}$ alkyl optionally substituted with aryl; and a compound of formula (VII):

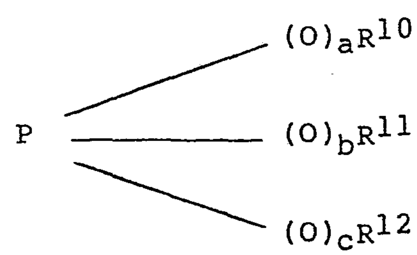

(VII)

wherein a, b and c are independently 0 or 1, and $R^{10}$, $R^{11}$ and $R^{12}$ are independently aryl or $C_{1-6}$ alkyl optionally substituted with aryl.

In the compounds of the formula (VI) $R^8$ and $R^9$ are preferably selected from methyl, ethyl, propyl, butyl, phenyl, and benzyl, the ethyl and t-butyl groups being preferred. Thus a preferred compound of formula (VI) is diethyl azodicarboxylate.

It is often convenient that $R^8$ and $R^9$ represent the same group.

Preferred compounds of the formula (VII) include triarylphosphines and trialkylphosphites. Preferred groups $R^{10}$, $R^{11}$ and $R^{12}$ include methyl, ethyl, n-propyl, n-butyl, benzyl, phenyl and methoxyphenyl. Conveniently, $R^{10}$, $R^{11}$ and $R^{12}$ represent the same group. A particularly preferred compound of the formula (VII) is triphenylphosphine.

Generally, approximately two equivalents of the compounds of the formulae (VI) and (VII) are used per mole of compound (V).

The elimination reaction is generally carried out at non-extreme temperature, such as -20°C to +100°C. We have found it convenient to begin the reaction at a depressed temperature, such as 0°C, and then to allow the temperature to rise to about room temperature.

The reaction is performed in an inert aprotic organic solvent. Suitable solvents include tetrahydrofuran, dioxane, ethyl acetate, benzene, dichloromethane and the like.

Alternatively, the dehydration process of this invention may be carried out by converting compound (V) into a compound of formula (VIII):

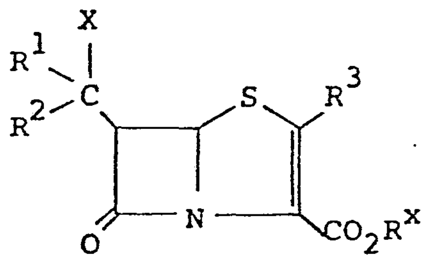

(VIII)

and eliminating the elements of a compound of formula H-X therefrom, wherein $R^1$, $R^2$, $R^3$ and $R^x$ are as defined with respect to formula (V) above, and X is a leaving group.

Suitable groups for the leaving group X include halogen; a group of formula $-O-SO_2(O)_n-R^{13}$ or $-O-CO-(O)_n-R^{13}$ wherein n is zero or 1 and $R^{13}$ is aryl or $C_{1-6}$ alkyl optionally substituted with aryl; and a group of formula $-OPO-(OR^{14})_2$ wherein $R^{14}$ is $C_{1-6}$ alkyl or aryl.

Preferred groups of formula $-OCO(O)_nR^{13}$ are those wherein n is zero and $R^{13}$ is $C_{1-6}$ alkyl, in particular acetoxy.

The elimination of the elements of a compound of the formula H-X is most conveniently brought about by treatment of a compound of the formula (VIII) with a base in an aprotic medium.

Suitable bases include powdered inorganic bases such as alkali metal carbonates, bicarbonates or hydroxides, for example powdered potassium carbonate, or alkali metal hydrides. Also suitable are organic bases of low nucleophilicity, for example,

1,8-diazabicyclo [5.4.0]undec-7-ene. Suitable solvents for use in this reaction include dimethylformamide, hexamethylphosphoramide, dichloromethane, tetrahydrofuran and the like.

The elimination may be effected at a low temperature such as -70 to +70°C, for example -40 to 0°C.

The compounds of formula (VIII) may be prepared from the compounds of formula (V) by replacing the hydroxy group by a group X. Alternatively the group X can be introduced into the molecule at an earlier stage in the synthesis of the penem nucleus. In particular the groups $-OSO_2(O)_nR^{13}$ and $-OCO(O)_nR^{13}$ can be introduced at the beginning of, or at any stage during, the synthesis of the penem. In each case, the group X is introduced by replacing a hydroxyl group. One particular intermediate useful for the synthesis of compound of formula (VIII) is the compound of formula (IX):

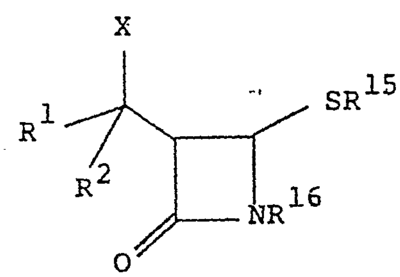

(IX)

wherein $R^1$ and $R^2$ are as defined with respect to formula (II) above, $R^{15}$ represents $C_{1-6}$ alkyl, aryl or aryl ($C_{1-6}$) alkyl, and $R^{16}$ represents hydrogen or an N-protecting group, and X is as hereinbefore defined.

Suitably $R^{15}$ is triphenyl methyl.

Suitable mild oxidising agents include perbenzoic acid, hydrogen peroxide, selenium dioxide or sodium metaperiodate. Suitable perbenzoic acids such as m-chloroperbenzoic acid are most preferred.

The compounds of formula (II) where $R^3$ represents $SR^a$ may also be prepared by a process which comprises reacting a sulphoxide of formula (X):

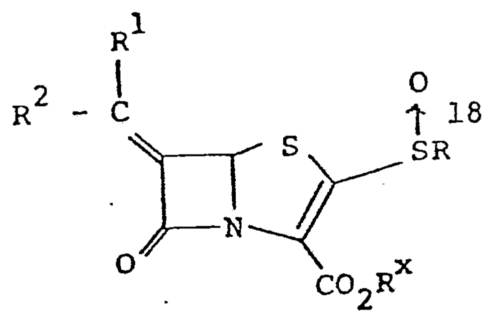

(X)

wherein $R^1$, $R^2$ and $R^x$ are as defined above and $R^{18}$ represents an organic radical different to the group $R^a$; with a thiol of formula (XI) or a reactive derivative thereof:

$$R^a - SH \qquad (XI)$$

and thereafter carrying out one or more of the following steps:

i) removal of any carboxyl blocking group $R^x$;

ii) converting the product to pharmaceutically acceptable salt or <u>in-vivo</u> hydrolysable ester group.

Suitable organic radicals for $R^{18}$ include those described above for $R^3$.

The process may be carried out in any inert solvent such as acetonitrile, N,N-dimethylformamide (DMF), tetrahydrofuran (THF), dioxane, hexamethylphosphoramide (HMPA) or glyme. The solvent DMF is preferred. A low temperature is preferred, suitably below 0°C, preferably below -20°C especially from about -30°C to about -70°C.

Alternatively we have found it useful to use a phase transfer catalyst. Particularly suitable phase transfer catalysts include tetra-n-butyl ammonium bromide, cetyldimethylbenzylammonium chloride and cetyltriethyl ammonium chloride. Suitable solvents include halogenated water immiscible solvents such as chloroform or dichloromethane in the presence of water.

When using a phase transfer catalyst, it is preferable to conduct the reaction between 0°C and ambient temperature.

When the thiol compound of formula (XI) itself is employed, the reaction is generally carried out in the presence of a base, although this is not essential. Examples of suitable bases include sodium hydride, potassium hydride, sodium amide, potassium amide, sodium hydroxide, potassium hydroxide, sodium ethoxide, soduim methoxide, potassium butoxide, triethylamine, tripropylamine and diisopropylethylamine. Advantageously, the base is used in an amount of at least 0.9 equivalent, preferably 1.0 to 1.2 equivalents, per mole of the thiol compound.

Instead of using the base in the reaction, a reactive derivative of the thiol compound of formula (XI) may be used. Preferably the reactive derivative

is a salt of the thiol (XI), in particular a salt with an alkali metal, preferably sodium or potassium.

The amount of the thiol compound of formula (XI) or its reactive derivative is not critical. Generally, however, it is used in an amount of at least 1.0 mole, preferably from 1 to 3, per mole of the compound of formula (X).

Compounds of formula (X) may be prepared by S-oxidation of a compound of formula (XII):

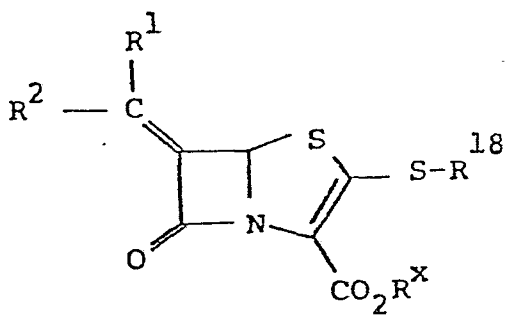

(XII)

wherein $R^1$, $R^2$, $R^{18}$ and $R^x$ are as defined with respect to formula (X) above; with a mild oxidising agent.

Suitable mild oxidising agents include perbenzoic acid, hydrogen peroxide, selenium dioxide or sodium metaperiodate. Suitable perbenzoic acids such as m-chloroperbenzoic acid are most preferred.

The present invention also provides a pharmaceutical composition which comprises a compound of this invention and a pharmaceutically acceptable carrier.

The compositions of the invention include those in a form adapted for oral, topical or parenteral use and may be used for the treatment of the infection in animals especially mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders,c olours, flavours, preservatives, disintegrant and the like in accordance with conventional pharmaceutical practice in the manner well understood by those skilled in the art of formulating antibiotics.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water; sterile saline or the like and may also contain suspending agents such as polyvinylpyrrolidone, lecithin or the like. For use in such suspensions the compounds of this invention should be in the form of fine particles.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

Alternatively, such compostions may be prepared in an acceptable oil suspending agent such as arachis oil or its equivalent. For use in such suspensions the

compounds of this invention should be in the form of fine particles.

Unit dose compositions comprising a compound of this invention adapted for oral administration form a further suitable composition aspect of this invention.

Unit dose compositions comprising a compound of this invention adapted for topical administration are also presented by this invention.

The compound of the formula may be present in the composition as sole therapeutic agent or it may be present together with other therapeutic agents such as, for example, a penicillin or cephalosporin. Considerable advantages accrue from the inclusion of a penicillin or cephalosporin which shows instability to β-lactamases since the resulting composition shows enhanced effectiveness (synergy). Suitable penicillins, cephalosporins or other β-lactam antibiotics for inclusion in such synergistic compositions include not only those known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, sulbenicillin, piperacillin, and other well known penicillins including pro-drugs thereof such as their in vivo hydrolysable esters such as the acetoxymethyl, pivaloyloxymethyl, α-ethoxycarbonyloxyethyl or phthalidyl esters of ampicillin benzylpenicillin or amoxycillin, and

aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

Suitable cephalosporins for inclusion in the compositions of this invention include, for example, cefatrizine, cephaloridine, cephalothin, cefazolin, cephalexin, cephacetrile, cephapirin, cephamandole nafate, cephradine, 4-hydroxycephalexin, cefaparole, cephaloglycin, cefoperazone, and other well known cephalosporins or pro-drugs thereof as well as newer broad spectrum cephalosporins such as cefotaxin and ceftazidine.

Such compounds are frequently used in the form of a salt of hydrate or the like.

Naturally if the penicillin or cephalosporin present in the composition is not suitable for oral administration then the composition will be adapted for parenteral administration.

Highly favoured penicillins for use in the compositions of this invention include ampicillin, amoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example, in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt, for example the sodium salt.

When present together with a cephalosporin or penicillin, the ratio of a compound of the invention to the penicillin or cephalosporin agent may vary over a wide range of ratios, such as from 10:1 to 1:10, for example about 3:1, 2:1, 1:1, 1:2, 1:3, 1:4, 1:5 or 1:6 (wt/wt, based on pure free antibiotic equivalent).

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of inter alia, the respiratory tract, the urinary tract and soft tissues in humans.

Normally for adult (70kg) human treatment between 50 and 3000 mg of the compounds of the invention will be administered each day of treatment. This corresponds to a dosage of 0.7 to 50mg/kg per day. More usually between 100 and 1000 mg of the compounds of the invention will be administered per day, corresponding to from 1.5 to 15 mg/kg per day, for example at 1-6 doses, more usually as 2, 3 or 4 doses. However for the treatment of more severe systemic infections or infections of particularly intransigent organisms higher doses may be used in accordance with clinical practice.

The penicillins or cephalosporin in the synergistic composition of this invention will normally be present at approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug thereof and from 25 to 500 mg of a compound of this invention.

Most suitably this form of composition will contain ampicillin trihydrate, ampicillin anhydrate, sodium ampicillin, hetacillin, pivampicillin, hydrochloride, bacampicillin hydrochloride or talampicillin hydrochloride. Most suitably this form of the composition will contain a compound of the formula (II) when in crystalline form.

Most suitably the preceding composition will contain from 200 to 700 mg of the penicillin component. Most suitably the preceding composition will comprise from 50 to 250 mg of a compound of the formula (II) preferably in crystalline form.

Such compositions may be adapted for oral or parenteral use except when containing an in vivo hydrolysable ester of ampicillin or amoxycillin in which case the compositions will not be adapted for parenteral administration.

Another particularly favoured compositions of this invention will contain from 200 to 2000 mg of carbenicillin, ticarcillin or a pro-drug thereof and from 50 to 500 mg of a compound of this invention.

Suitably this form of composition will contain di-sodium carbenicillin. Suitably this form of the composition will contain di-sodium ticarcillin.

More suitably this form of the composition will contain from 75 to 250 mg of a compound of the formula (II) preferably in crystalline form. Such compositions containing di-salts of carbenicillin and ticarcillin will be adapted for parenteral administration.

The present invention also provides a method of treating bacterial infections in animals, in particular humans or domestic mammals, which comprises the administration of a composition of this invention

Commonly the infection treated will be due to a strain of Staphylococcus aureus, Klebsiella aerogenes, Escherichia coli, Proteus sp., Bacteroides fragilis or the like. The organisms believed to be most readily treated by an antibacterially effective amount of a compound of this invention is Staphylococcus aureus. The other organisms named are more readily treated by

using a synergistically effective amount of the compound of this invention and a penicillin or cephalosporin. The administration of the two components may take place separately but in general we prefer to use a composition containing both the synergist and the penicillin or cephalosporin.

The indications for treatment include respiratory tract and urinary tract infections in humans.

The following Examples illustrate this invention.

Preparation 1(a)

1-t-Butyldimethylsilyl -3- [(2-furyl) hydroxymethyl] -4-tritylthioazetidin-2-one

A solution of n-butyl lithium (2.5M in hexane, 9.6 ml) was added to a stirred solution of diisopropylamine (3.35 ml) in dry tetrahydrofuran (THF) (100 ml) at -30°C under dry argon. After stirring at -30°C for 10 minutes the mixture was cooled to -76°C and treated, dropwise over 5 minutes, with a solution of 1-t-butyldimethylsilyl -4-tritylthio azetidin-2-one (1) (9.18g) (Bristol-Myers Patent GB2042515A) in dry THF (50 ml). After a further 15 minutes at -76°C the stirred mixture was treated, dropwise over 5 minutes, with a solution of furfuraldehyde (2.50g) in dry THF (10 ml). After 20 minutes at -76°C the stirred mixture was treated with saturated aqueous ammonium chloride solution (50 ml) and was allowed to attain room temperature. The mixture was diluted with ethyl acetate (500 ml) and washed with brine (3 x 50 ml). The dried ($MgSO_4$) organic layer was evaporated and the residue chromatographed on silica gel eluting with ethyl acetate/hexane mixtures to give three fractions. The least polar fraction (2.48g) contained an approximately 2:1 mixture of trans (Isomer A) and cis (Isomer B) isomers of the title azetidinones (2); ν max ($CHCl_3$) 3600-3200, 1745 $cm^{-1}$; δ ppm ($CDCl_3$) 0.22 and 0.23 (4H, each s, $SiMe_2$ Isomer A), 0.36 and 0.37 (2H, each/s $SiMe_2$ Isomer B), 0.91 and 0.97 (9H, each s), 1.50-1.70 br (⅔H,m, exch.$D_2O$), 2.70-2.85 br (⅓H,m,exch.$D_2O$), 3.45-3.55 (1⅓H,m), 3.61-3.76 br (⅔H,m,collapses to 3.68,d,J3Hz on

exch.$D_2O$), 4.43 (⅔H,d,J2Hz), 4.79 (⅓H,d,J4½Hz), 6.19-6.33 (2H,m), 7.10-7.60 (1/6H,m). The second fraction (6.31g) contained a pure <u>trans</u>-azetidinone (2) (Isomer C), ν max ($CHCl_3$) 3600-3150, 1735 $cm^{-1}$; δ ppm ($CDCl_3$) 0.09 (3H,s), 0.83 (9H,s), 2.58br(1H,d,J9Hz),3.70(1H,dd,J7.0and 1.7Hz), 3.80-4.10br (1H,m,collapses to d, J 7.0Hz on irradiation at 2.58 ppm), 3.99 (1H,d,J1.7Hz), 5.92-6.00 (1H,m), 6.19-6.27 (1H,m), 7.15-7.50 (16H,m), 1 MeSi signal was obscured by the TMS signal; (Found: $[MNH_2Et_2]^+$, $^m/_e$ 629, $Et_2NH$ CI). The most polar fraction (444mg) contained a pure <u>cis</u> - azetidinone (2) (Isomer D), ν max ($CHCl_3$) 3600-3200, 1740 $cm^{-1}$., δ ppm ($CDCl_3$) 0.88 (9H,s), 1.85-2.25 (1H, broad signal, exch $D_2O$), 3.64 (1H,dd,J4.8 and 8.0Hz), 4.30-4.60 (2H,m,collapses to 4.38,d,J4.8z and 4.39,d,J8.0Hz on exch $D_2O$), 6.15-6.35 (2H,m), 7.20-7.60 (16H,m), both $SiMe_2$ signals were obscured by the TMS signal; (Found: $[MNH_2Et_2]+$, $^m/_e$ 629, $Et_2NH$ CI).

The compounds described in Preparations 1(b) to 3(b) and Examples 1(a) to 3(b) are derived from Isomer C.

<u>Preparation 1(b)</u>

<u>(3RS,4SR) 3- [Acetoxy (2-furyl) methyl] -1-t-butyldimethylsilyl-4-tritylthioazetidin-2-one</u>

The <u>trans</u>-azetidinone (2) (Isomer C) (111 mg) from Preparation 1(a) was dissolved in dry dichloromethane (4ml), cooled in an ice bath, and treated with 4-dimethylaminopyridine (2mg), triethylamine (24mg) and acetic anhydride (24mg). After stirring at room temperature for 4 hours the mixture was diluted with ethyl acetate (20ml) and washed with 5% citric acid (2ml), brine (2ml), saturated $NaHCO_3$ (2ml) and brine (2ml). The dried ($MgSO_4$) organic layer was evaporated and the residue

chromatographed on silica gel eluting with ethyl acetate/ hexane mixtures to give the title acetate (3) (103mg) as a gum, ν max ($CHCl_3$) 1745 $cm^{-1}$; δ ppm ($CDCl_3$) -0.04 (3H,s), 0.07 (3H,s), 0.82 (9H,s), 1.93 (3H,s), 3.76-3.90 (2H,m), 5.17 (1H,d,J7Hz), 6.02 (1H,d,J3Hz), 6.21 (1H,dd,J2 and 3Hz), 7.12-7.50 (16H,m); (Found: $[MNH_2Et_2]^+$, $^m/_e$ 671, $Et_2NH$ CI).

Preparation 1(c)

(3RS,4SR) 3-[Acetoxy (2-furyl) methyl] - 4 - tritylthioazetidin -2-one

A solution of the acetate (3) (58 mg) from Preparation 1(b) in methanol (3 ml) was cooled to -20°C and treated with a solution of potassium fluoride (6.4 mg) in methanol (0.5 ml). After stirring for 45 minutes at -20°C the mixture was evaporated. The residue was dissolved in ethyl acetate (25 ml) and washed with brine (3 x 2 ml). The dried ($MgSO_4$) organic layer was evaporated and the residue chromatographed on silica gel eluting with ethyl acetate/ hexane mixtures to give the title azetidinone (4) (40 mg) as a solid, m.p. 167-168°C (rods ex ethyl acetate/ hexane); ν max ($CHCl_3$) 3390, 1770 $cm^{-1}$; δ ppm ($CDCl_3$) 2.02 (3H,s), 3.63 (1H, ddd, J5.5, 2.7 and 1.0 Hz), 4.21 br (1H,s), 4.52 (1H,d,J2.7Hz), 6.14 (1H,d,J 5.5 Hz), 6.35-6.40 (2H,m), 7.14-7.50 (16H,m); (Found: C,72.1; H,5.5; N,2.8; S,6.9. $C_{29}H_{25}NO_4S$ requires C, 72.1; H,5.2; N, 2.9; S,6.6%).

Preparation 1(d)

(3RS, 4SR) 3-[Acetoxy (2-furyl) methyl]-1- (1-p-nitrobenzyloxycarbonyl-1-triphenylphosphoranylidenemethyl) -4-tritylthioazetidin-2-one

The azetidinone (4) (483 mg) from Preparation 1(c) and p-nitrobenzyl glyoxylate monohydrate (250 mg) were heated in refluxing benzene (20 ml) with provision for the azeotropic removal of water (Dean and Stark apparatus containing molecular sieves 4A) for 1 hour. The mixture was cooled to room temperature and treated with triethylamine (10 mg). After stirring at room temperature for 40 minutes the mixture was evaporated to give a crude hydroxyester (5) as a gum, ν max ($CHCl_3$) 3500 br, 1775, 1760 sh. $cm^{-1}$. A solution of the crude hydroxyester (5) in dry THF (15 ml) was cooled to -10°C and treated with 2,6- lutidine (160 mg) and thionyl chloride (178 mg). After stirring at -10°C for 10 minutes the mixture was filtered and evaporated. The residue was re-evaporated from dry toluene (2 x 3 ml) to give a crude chloroester (6) as a gum, ν max ($CHCl_3$) 1780 $cm^{-1}$. A mixture of the crude chloroester (6), triphenylphosphine (1.05 g) and 2,6-lutidine (128 mg) were heated in dry dioxan (20 ml) at 100°C under dry argon for 15 hours. The mixture was diluted with ethyl acetate (100 ml) and washed with N. hydrochloric acid (5 ml), brine (5 ml), saturated $NaHCO_3$ (5 ml) and brine (3 x 5 ml). The dried ($MgSO_4$) organic layer was evaporated and chromatographed on silica gel eluting with ethyl acetate/hexane mixtures to give the title phosphorane (7) (378 mg) as yellow amorphous solid, ν max ($CHCl_3$) 1750, 1615, 1605 sh.$cm^{-1}$.

Preparation 1(e)

(3RS, 4SR) Silver 3-[Acetoxy (2-furyl) methyl] -1- (1-p-nitrobenzyloxycarbonyl-1-triphenylphosphoranylidenemethyl) azetidin-2-one-4-thiolate

The phosphorane (7) (378 mg) from Preparation 1(d) was dissolved in methanol (8 ml) and treated with pyridine (41 mg) and silver nitrate (3.5 ml of a 0.15 M. solution in methanol). After 30 minutes at room temperature the stirred mixture was cooled in an ice bath for 10 minutes and filtered. The residue was washed with cold methanol (2 ml) and dry ether (3 x 2 ml) and dried under vacuum to give the title silver thiolate (8) (270 mg) as a buff coloured solid, ν max (Nujol) 1760 br., 1600 br.$cm^{-1}$.

Preparation 1(f)

(5RS, 6SR) p-Nitrobenzyl 6- [Acetoxy (2-furyl) methyl] penem -3- carboxylate

A stirred, ice bath cooled, suspension of the silver thiolate (8) from Preparation 1(e), (200 mg) in dry dichloromethane (5 ml) was treated with acetic-formic anhydride (0.2 ml), 4-dimethylamino pyridine (27 mg), and triethylamine hydrochloride (343 mg). The ice bath was removed and the mixture stirred for a further 10 minutes. The mixture was filtered through Kieselguhr and the residue was washed with ethyl acetate (20 ml). The combined filtrates were washed with 5% citric acid (5 ml), brine (5 ml), saturated $NaHCO_3$ (5 ml) and brine (3x5 ml). The dried ($MgSO_4$) organic layer was heated under argon at 50°C for 30 minutes. The mixture was evaporated and the residue chromatographed on silica gel eluting with ethyl

acetate/hexane mixtures to give the title penem (9) (90 mg) as a solid, m.p. 146-148°C (plates ex ethyl acetate/hexane); λ max (EtOH) 262 (Em 13,050) and 315 nm (8580); ν max ($CHCl_3$) 1795, 1745, 1720 $cm^{-1}$; δ ppm ($CDCl_3$) 2.07 (3H,s), 4.34 (1H, ddd, J 4.3, 2.0 and 1.0Hz), 5.22 and 5.43 (2H, ABq, J14Hz), 6.02 (1H,d,J2.0Hz), 6.24 (1H,d,J4.3Hz), 6.3 - 6.5 (2H,m), 7.31 (1H,d,J1.0Hz), 7.35 - 7.45 (1H,m), 7.57 (2H,d,J9Hz), 8.21 (2H,d,J9Hz); (Found: C,54.4; H,3.7; N,6.3; S,7.4. $C_{20}H_{16}N_2O_8S$ requires C,54.0; H,3.6; N,6.3; S,7.2%).

Example 1(a)

(5 RS) p-Nitrobenzyl (Z)- 6- Furfurylidenepenem-3-carboxylate

A solution of 1,8-diazabicyclo [5.4.0] undec-7-ene (DBU) (61 mg) in dry dichloromethane (1 ml) was added, dropwise over 1 minute, to a stirred solution of the penem (9) (118 mg) from Preparation 1(f) in dry dichloromethane (5 ml) at -40°C. The mixture was stirred at -40°C for 10 minutes during which a yellow solid precipitated. The solid was redissolved by addition of dichloromethane (20 ml) and the mixture was washed with 5% citric acid (3 ml), brine (3 ml), saturated $NaHCO_3$ (3 ml) and brine (3 x 3 ml). The dried ($MgSO_4$) organic layer was evaporated and the residue chromatographed on silica gel eluting with dichloromethane/ethyl acetate mixtures to give the title penem ester (10) (96 mg) as an orange-yellow solid, m.p. 181-184°C (microcrystalline ex ethyl acetate/hexane); λ max (EtOH) 265 (Em 11,710) and 315 nm (31,250); ν max ($CHCl_3$) 1780, 1720, 1670 $cm^{-1}$;

δ ppm ($CDCl_3$) 5.29 and 5.47 (2H,ABq, J 13Hz), 6.56 (1H,dd,J2 and 4Hz), 6.59 (1H,d,J 1.5Hz), 6.74 (1H,d,J4Hz), 6.96 (1H,br.s, sharpens to d, J 1.5 Hz on irradiation at 7.62 ppm), 7.38 (1H,s), 7.58-7.65 (3H,m), 8.24 (2H,d, J9Hz); (Found: C,56.1; H, 3.3; N, 7.2; S,9.0. $C_{18}H_{12}N_2O_6S$ requires C,56.3; H, 3.1; N, 7.3; S, 8.3%).

Example 1(b)

(5 RS) Sodium (Z) -6- Furfurylidenepenem - 3 - carboxylate

The penem ester (1) (30 mg) from Example 1(a) was dissolved in a mixture of dioxan (8 ml) and water (2 ml) and was hydrogenated over 5% palladium/charcoal catalyst (45 mg) at S.T.P. for 40 minutes. Further catalyst (30 mg) was added and the hydrogenation continued for 40 minutes. A 1% sodium bicarbonate solution (0.66 ml) was added and the mixture filtered through Kieselguhr the residue being washed with a little aqueous dioxan. The combined filtrates were evaporated and the residue chromatographed on Biogel P2 eluting with water. The appropriate fractions were evaporated and the residue re-evaporated from ethanol (1 ml) and dry toluene (2 x 1 ml) to give, after trituration with dry ether, the title sodium salt (11) (9.5 mg) as an orange coloured amorphous solid; λ max ($H_2O$) 308 nm (Em 21,020); δ ppm ($D_2O$) 6.61 (1H, dd, J 3.5 and 2.0 Hz), 6.64 (1H,s), 6.89 (1H,d,J 3.5 Hz), 7.06 (1H,s), 7.08 (1H,s), 7.73br (1H,d, J approx 2Hz).

Preparation 2(a)

(3RS, 4SR) 3-[Acetoxy (2-furyl) methyl]- 4 - ethylthiothiocarbonylthio - 1 - (1-p-nitrobenzyloxycarbonyl - 1 - triphenylphosphoranylidenemethyl azetidin - 2 - one

A stirred, ice bath cooled, suspension of the silver thiolate (8) (200 mg) from Preparation 1(e) in dry dichloromethane (5 ml) was treated with pyridine (59 mg) and ethyl dithiochloroformate (70 mg). The ice bath was removed and the mixture was stirred for 1 hour. The mixture was filtered through Kieselguhr and the residue was washed with ethyl acetate (25 ml). The combined filtrates were washed with 5% citric acid (3 ml), brine (3 ml), saturated $NaHCO_3$ (3 ml) and brine (3 x 3ml). The dried ($MgSO_4$) organic layer was evaporated and the residue chromatographed on silica gel eluting with ethyl acetate/hexane mixtures to give the title phosphorane (12) (134 mg) as a yellow amorphous solid; ν max ($CHCl_3$) 1765, 1625, 1610 sh. $cm^{-1}$.

Preparation 2(b)

(5RS, 6SR) and (5RS, 6RS) p-Nitrobenzyl 6-[Acetoxy (2-furyl) methyl] -2- ethylthiopenem -3- carboxylate

The phosphorane (12) (132 mg) from Preparation 2(a) was heated in refluxing xylene (65 ml) under argon for 7½ hours. The mixture was cooled, evaporated and the residue chromatographed on silica gel eluting with dichloromethane/ethyl acetate mixtures to give two title products. The less polar product the <u>cis</u> - penem ester

(14) (5 mg) was obtained as an amorphous solid; λ max (EtOH) 259 (Em 15880) and 333 nm (10,520); ν max ($CHCl_3$) 1800, 1750, 1695 $cm^{-1}$; δ ppm ($CDCl_3$) 1.41 (3H,t,J7$H_2$), 2.08 (3H,s), 2.91-3.17 (2H,m),4.72 (1H,dd,J4.0 and 10.0 Hz), 5.19 and 5.43 (2H, ABq,J13Hz), 5.86 (1H,d,J 4.0Hz), 6.27 (1H,d,J10.0Hz), 6.42 (1H,dd,J3 and 2Hz), 6.55 (1H,d,J3Hz), 7.48 (1H,d,J2Hz), 7.58 (2H,d,J9Hz), 8.20 (2H,d,J9Hz); (Found: $MNH_4{}^+$, $^m/_e$ 522, $NH_3$CI). The more polar product the trans-penem ester (13) (25 mg) was obtained as an amorphous solid; λ max (EtOH) 259 (Em 15890) and 337 nm (10,095); ν max ($CHCl_3$) 1795, 1745, 1695 $cm^{-1}$; δ ppm ($CDCl_3$) 1.39 (3H,t,J7Hz), 2.09 (3H,s), 2.88-3.12 (2H,m), 4.29 (1H,dd,J4.5 and 2.0Hz), 5.26 and 5.46 (2H, ABq, J12Hz), 5.95 (1H,d,J2.0Hz)6.28 (1H,d,J4.5Hz), 6.40 (1H,dd,J2 and 3Hz), 6.47 (1H,d,J3Hz), 7.41 (1H,d,J2Hz), 7.61 (2H,d,J8Hz), 8.22 (2H,d,J8Hz); (Found: $[MNH_4]^+$, $^m/_e$ 522, $NH_3$ CI).

In a second experiment the phosphorane (12) (1.365g) was heated in refluxing xylene (700 ml) containing hydroquinone (100 mg) under argon for 10 hours. Work up as described above gave the cis-penem (14) (136mg) and the trans-penem (13) (465 mg).

Example 2(a)

(5RS) p-Nitrobenzyl 2-Ethylthio -6- furfurylidenepenem -3- carboxylate

A solution of DBU (27 mg) in dry dichloromethane (1 ml) was added, dropwise over 1 minute, to a stirred solution of a mixture of the penem esters (13) and (14) (60 mg) from Preparation 2(b) in dry dichloromethane (3 ml) at -40$^oC$. After 10 minutes at -40$^oC$ the mixture was worked up as for Example 1(a) to give a mixture of (Z) and (E) isomers of the title penem (15) (53 mg) which on trituration with ether gave the (Z)-isomer (36 mg) as a yellow solid; λ max (EtOH) 260 (Em 16370) and 319 nm (36,060); ν max

($CHCl_3$) 1775, 1690 sh, 1670 $cm^{-1}$; δ ppm ($CDCl_3$) 1.36 (3H,t,J7Hz), 2.75-3.20 (2H,m), 5.21 and 5.50 (2H, ABq, J14Hz), 6.44 (1H,s), 6.52 (1H,dd,J2 and 3Hz), 6.70 (1H,d,J3Hz), 6.90 (1H,s), 7.56-7.70 (3H,m), 8.21 (2H,d,J9Hz); (Found: $M^+$, 444.0460. $C_{20}H_{16}N_2O_6S_2$ requires M,444.0448).

Example 2(b)

(5RS) Sodium 2-Ethylthio - (Z)-6-furfurylidenepenem -3-carboxylate

The penem ester (15) (Z-isomer) (34 mg) from Example 2(a) was dissolved in a mixture of dioxan (8 ml) and water (2 ml) and was hydrogenated over 5% palladium/charcoal catalyst (51 mg) at S.T.P. for 40 minutes. Further catalyst (34 mg) was added and the hydrogenation continued for 40 minutes. A 1% sodium bicarbonate solution (0.64 ml) was added and the mixture was worked up as for Example 1(b) to give the title sodium salt (16) (7.6 mg) as an orange-yellow amorpous solid; λ max ($H_2O$) 238 (Em 7420) and 315 nm (24,770).

Preparation 3(a)

(5 RS, 6SR) p-Nitrobenzyl 6- [Acetoxy (2-furyl)methyl] -2- ethylsulphinylpenem -3- carboxylate

A solution of m-chloroperbenzoic acid (16.5 mg) in dichloromethane (1 ml) was added in one portion to a stirred, ice bath cooled, mixture of the trans-penem (13) (37 mg) from Preparation 2(b), dichloromethane (2 ml) and saturated sodium bicarbonate solution (2 ml).

The mixture was stirred vigorously at ice bath temperature for 10 minutes and diluted with dichloromethane (10 ml). The organic layer was separated and washed with brine (3 x 2 ml). The dried ($MgSO_4$) organic layer was evaporated and the residue chromatographed on silica gel eluting with ethyl acetate/hexane mixtures to give the title sulphoxide (17) (20 mg), an approximately 1:1 mixture of isomers, as an amorphous solid; $\nu$ max ($CHCl_3$) 1800, 1745, 1705 $cm^{-1}$; $\delta$ ppm ($CDCl_3$) 1.40 (3H,t,J7Hz), 2.05 (3H,s), 2.90-3.25(2H,m), 4.43 (1H,dd, J2 and 4Hz), 5.22 and 5.45 (2H,ABq,J14Hz), 6.00 (½H,d,J2Hz), 6.10 (½H,d,J2Hz), 6.24 (1H,d,J4Hz), 6.31-6.49 (2H,m), 7.39br (1H,s), 7.57 and 7.59 (2H,each d, J8Hz), 8.22 (2H,d J8Hz).

Preparation 3(b)

(5RS,6SR) p-Nitrobenzyl 6- [Acetoxy (2-furyl) methyl] -2-(2-hydroxyethylthio) penem -3- carboxylate

A solution of diisopropylethylamine (5 mg) in dry acetonitrile (0.5 ml) was added, dropwise over 0.5 minute, to a stirred solution of the sulphoxide (17) from Preparation 3(a) (20 mg) and 2-mercaptoethanol (6 mg) in dry acetonitrile (1 ml) at -40°C. After stirring at -40°C for 10 minutes the mixture was diluted with ethyl acetate (10 ml) and washed with 5% citric acid (2 ml), brine (2 ml), saturated $NaHCO_3$ (2 ml), and brine (3 x 2 ml). The dried ($Mg SO_4$) organic layer was evaporated and the residue chromatographed on silica gel eluting with ethyl acetate/hexane mixtures to give the title penem ester (18) (10 mg) as an amorphous solid; $\nu$ max ($CHCl_3$) 3600-3100, 1790, 1740, 1690 $cm^{-1}$; $\delta$ ppm

($CDCl_3$) 2.02 (1H,t,J5.9Hz, collapses to S on irradiation at 3.9 ppm), 2.09 (3H,s), 3.12 (1H,dt, J13.9 and 6.2Hz, collapses to d,J 13.9Hz on irradiation at 3.9 ppm), 3.22 (1H,dt,J13.9 and 6.0Hz, collapses to d, J13.9Hz on irradiation at 3.9 ppm), 3.85-3.95 (2H,m), 4.30 (1H,dd, J4.5 and 1.8Hz), 5.26 and 5.46 (2H,ABq, J13.8Hz), 5.96 (1H,d,J1.8 Hz), 6.28 (1H,d,J4.5Hz), 6.39 (1H,dd,J1.9 and 3.4 Hz), 6.48 (1H,d,J3.4Hz), 7.41 (1H,d,J 1.9Hz), 7.62 (2H,d,J 8.7Hz), 8.22 (2H,d,J8.7Hz).

Example 3(a)

(5RS) p-Nitrobenzyl (z) -6-Furfurylidene -2-(2-hydroxyethylthio) penem-3-carboxylate

A solution of DBU (29 mg) in dry dichloromethane (1 ml) was added, dropwise over 1 minutes, to a stirred solution of the penem ester (18) (67 mg) from Preparation 3(b) in dry dichloromethane (3 ml) at -40°C. After stirring at -40°C for 10 minutes the mixture was worked up as Example 1(a) to give the title penem (19) (54 mg), mp 157-159°C (fine needles ex ethyl acetate/hexane); λ max (EtOH) 262 (Em 15900) and 320nm (35020); ν max ($CHCl_3$) 1770, 1670 $cm^{-1}$; δ ppm ($CDCl_3$) 1.93 (1H,t,J6.2Hz), 3.08-3.26 (2H,m), 3.84-3.92 (2H,m), 5.26 and 5.50 (2H,ABq, J13.8 Hz), 6.46 (1H,d,J1.0Hz), 6.56 (1H,dd, J3.5 and 1.9Hz, 6.75 (1H,d,J3.5Hz), 6.94 (1H, slightly broadeneds), 7.61 br (1H,d,J1.6Hz), 7.67 (2H,d,J8.8Hz), 8.24 (2H,d,J8.8Hz). (Found: C,52.1; H,3.8; N,6.0; S,13.4. $C_{20}H_{16}N_2O_7S_2$ requires C,52.2; H,3.5; N,6.1; S,13.9%).

Example 3(b)

(5RS) Sodium (Z)-6-Furfurylidene -2-(2-hydroxyethylthio) penem -3- carboxylate

The penem ester (19) (48 mg) from Example 3(a) was dissolved in a mixture of dioxan (8 ml) and water (2 ml) and hydrogenated over 5% palladium/charcoal catalyst (72 mg) at S.T.P. for 45 minutes. A 1% sodium bicarbonate solution (0.88 ml) was added and the mixture was worked up as for Example 1(b) to give the title sodium salt (20) (18.1 mg) as an orange coloured amorphous solid; λ max ($H_2O$) approx 370 (inflection Em3940), 314 (23600), 236 nm (7380); δ ppm ($D_2O$) 2.94-3.20 (2H,m), 3.88-3.95 (2H,m), 6.52 (1H,s), 6.63 (1H,dd,J3 and 1½Hz), 6.90 (1H,d,J3Hz), 7.08 (1H,s), 7.74 br (1H,s).

Preparation 4(a)

3- [(5-Acetoxymethyl-2-furyl) hydroxymethyl] -1-t-butyldimethylsilyl -4- tritylthioazetidin -2-one

A solution of n-butyl lithium (2.5 M. in hexane, 0.48 ml) was added to a stirred solution of diisopropylamine (0.17 ml) in dry THF (8 ml) at -30°C under dry argon. The mixture was stirred at -30° for 10 minutes, cooled to -76°C, and treated with a solution of the azetidinone (1) (459 mg) used in Preparation 1(a) in dry THF (4 ml). After a further 15 minutes at -76°C the stirred mixture was treated with a solution of 5-acetoxymethyl-2-furaldehyde (185 mg) in dry THF (1 ml). The mixture was stirred at -76°C for a further 20 minutes and worked up as for Preparation 1(a) to give two fractions. The less polar fraction (104 mg) contained an approximately 1:1 mixture of trans (Isomer A) and cis (Isomer B) isomers of the title azetidinone (21); ν max ($CHCl_3$) 3600-3200,

1740 $cm^{-1}$; δ ppm ($CDCl_3$) 0.95 (9H,s), 1.67 (½H,d,J6Hz, exch.$D_2O$), 2.04 (3H,s), 2.78 (½H,d,J 6Hz,exch.$D_2O$), 3.40-3.70 (2H,m, simplifies on exch.$D_2O$), 4.45 (½H,d,J 1½Hz), 4.78 (½H,d,J5Hz), 4.95 (2H,s), 6.20-6.30 (2H,m), 7.10-7.60 (15H,m), the $SiMe_2$ signals were obscured by the TMS signal. The more polar fraction (168 mg) contained a pure trans-azetidinone (21) (Isomer C), m.p. 121-123°C (rhombs ex ethyl acetate/hexane); ν max ($CHCl_3$) 3600-3200, 1740 $cm^{-1}$; δ ppm ($CDCl_3$) 0.82 (9H,s), 2.00 (3H,s), 2.54 (1H, broad signal, exch.$D_2O$), 3.69 (1H,dd,J2 and 6Hz), 3.85-4.05 br (1H,m,collapses to 3.93,d,J6Hz on exch. $D_2O$), 4.10 (1H,d,J 2Hz), 4.88 (2H,s), 5.99 (1H,d, J3Hz), 6.24 (1H,d,J3Hz), 7.10-7.60 (15H,m), the $SIMe_2$ signals were obscured by the TMS signal; (Found: C,68.7; H,6.6; N,2.2; S,5.1 $C_{36}H_{41}NO_5SSi$ requires C,68.9; H,6.5; N,2.2; S,5.1%).

The compounds described in Preparations 4(b) to 6(e) and Examples 4(a) to 6(b) are derived from Isomer C from Preparation 4(a).

Preparation 4(b)

(3RS,4SR) 3- Acetoxy (5-acetoxymethyl-2-furyl) methyl -1-t-butyldimethylsilyl-4-tritylthioazetidin-2-one

The trans-azetidinone (21) (Isomer C) (180 mg), from Preparation 4(a) 4-dimethylaminopyridine (3 mg), triethylamine (35 mg), and acetic anhydride (35 mg) were stirred in dry dichloromethane (5 ml) at room temperature for 6 hours. The mixture was worked up as for Preparation 1(b) to give the title diacetate (22) (114 mg) as a solid, m.p. 111-112°C (chunks ex ethyl acetate/hexane); ν max

($CHCl_3$) 1745 $cm^{-1}$; δ ppm ($CDCl_3$) -0.4 and 0.6 ($SiMe_2$), 0.82 (9H,s), 1.91 (3H,s), 2.00 (3H,s), 3.83 (1H,dd,J2.0 and 6.1 Hz), 3.93 (1H,d,J2.0Hz), 4.89 (2H,s), 5.18 (1H,d,J6.1Hz), 6.04 (1H,d,J3Hz), 6.24 (1H,d,J3Hz), 7.10-7.50 (15H,m). (Found: C,68.2; H,6.6; N,2.1; S,4.9. $C_{38}H_{43}NO_6SSi$ requires C,68.2; H,6.4; N,2.1; S,4.8%).

Preparation 4(c)

(3RS,4SR) 3- [Acetoxy (5-acetoxymethyl-2-furyl) methyl] -4- tritylthioazetidin-2-one

A mixture of the diacetate (22) (90 mg) from Preparation 4(b), anhydrous potassium fluoride (16 mg) and 18-crown-6 (7 mg) was stirred in dry THF (3 ml) at room temperature for 22 hours. The mixture was diluted with ethyl acetate (15 ml) and washed with brine (3 x 3 ml). The dried ($MgSO_4$) organic layer was evaporated and the residue chromatographed on silica gel eluting with ethyl acetate/hexane to give the title azetidinone (23) (67 mg) as a solid, m.p. 133-134°C (rhombs ex ethanol/hexane); ν max ($CHCl_3$) 3375, 1770, 1740 $cm^{-1}$; δ ppm ($CDCl_3$) 1.97 (3H,s), 2.05 (3H,s), 3.69 (1H,dd,J3 and 6Hz), 4.25 br(1H,s), 4.49 (1H,d,J3Hz), 5.04 (2H,s), 6.14 (1H,d,J6Hz), 6.33 and 6.40 (2H, each d, J3½Hz), 7.20-7.50 (15H,m); (Found: C,69.2; H,5.4; N,2.4; S,6.1. $C_{32}H_{29}NO_6S$ requires C,69.2; H,5.2; N,2.5; S,5.8%).

Preparation 4(d)

(3RS,4SR) 3-[Acetoxy (5-acetoxymethyl-2-furyl) methyl] -1- (1-p-nitrobenzyloxycarbonyl-1-triphenylphosphoranylidenemethyl)-4-tritylthioazetidin-2-one

The azetidinone (23) (110 mg) from Preparation 4(c) and p-nitrobenzyl glyoxylate monohydrate (50 mg) were heated in refluxing benzene (5 ml) followed by treatment with triethylamine (2 mg) as for Preparation 1(d) gave a hydroxyester (24) as a crude solid; ν max ($CHCl_3$) 3600-3200, 1770, 1745 $cm^{-1}$. The crude hydroxyester (24) was treated with 2,6-lutidine (32 mg) and thionyl chloride (36 mg) in dry THF (5 ml) at -10°C as for Preparation 1(d) to give a crude chloroester (25) as a gum, max ($CHCl_3$) 1780, 1745 $cm^{-1}$. The crude chloroester (25), triphenylphosphine (210 mg) and 2,6-lutidine (24 mg) were heated in dry dioxan (5 ml) under dry argon at 90°C for 18 hours and then at 100°C for a further 8 hours. Work up of the mixture as for Preparation 1(d) gave the title phosphorane (26) (60 mg) as a yellow amorphous solid; ν max ($CHCl_3$) 1750, 1615, 1605 sh.$cm^{-1}$.

Preparation 4(e)

(3RS,4SR) Silver 3-[Acetoxy (5-acetoxymethyl-2-furyl) methyl]-1-(1-p-nitrobenzyloxycarbonyl-1-triphenylphosphoranylidenemethyl) azetidin-2-one-4-thiolate

The phosphorane (26 (400 mg) from Preparation 4(d) was dissolved in methanol (8 ml) and treated with pyridine (41 mg) and silver nitrate (3.4 ml of a 0.15 M. solution in methanol) as for Preparation 1(e) to give the title silver salt (27) (312 mg) as an amorphous solid; ν max ($CHCl_3$) 1740, 1600 $cm^{-1}$.

Preparation 4(f)

(5RS,6SR) p-Nitrobenzyl 6- [Acetoxy (5-acetoxymethyl-2-furyl) methyl] penem -3-carboxylate

A stirred, ice bath cooled, suspension of the silver thiolate (27) (200 mg) from Preparation 4(e) in dry dichloromethane (5 ml) was treated with acetic-formic anhydride (o.18 ml), 4-dimethylaminopyridine (25 ml), and triethylamine hydrochloride (315 mg) followed by heating the product as for Preparation 1(f) to give the title penem (28) (90 mg) as an amorphous solid; λ max (EtOH) 262 (Em 13100) and 314 nm (10,845); ν max ($CHCl_3$) 1795, 1740 br.$cm^{-1}$; δ ppm ($CDCl_3$) 2.07 (6H,s), 4.33 (lH,ddd,J 4.2,2.1 and approx lHz), 5.00 (2H,s), 5.22 and 5.43 (2H, ABq,J14Hz), 6.05 (lH,d,J 2.lHz), 6.21 (lH,d,J4.2Hz), 6.35 and 6.41 (2H, each d, J3½Hz), 7.30 (lH,d,J approx lHz), 7.56 (2H,d,J9Hz), 8.21 (2H,d,J9Hz).

Example 4(a)

(5RS) p-Nitrobenzyl (Z)-6- (5-Acetoxymethylfurfurylidene) penem -3- carboxylate

A solution of the penem (28) (97 mg) from Preparation 4(f) in dry dichloromethane (5 ml) was treated with DBU (43 mg) at -40°C as for Example 1(a) to give the title penem (29) (72 mg) as a yellow solid, m.p. 140-142°C (fine needles ex ethyl acetate/hexane); λ max (EtOH) 262 (Em 12700) and 317 nm (34940); ν max ($CHCl_3$) 1785, 1745, 1720, 1670 $cm^{-1}$; δ ppm ($CDCl_3$) 2.10 (3H,s), 5.07 (2H,s), 5.25 and 5.47 (2H,ABq, J14Hz), 6.50 and 6.67 (2H, each d, J 3½Hz), 6.57 (1H,s), 6.89 (1H,s), 7.36 (1H,s), 7.59 (2H,d, J9Hz), 8.21 (2H,d,J 9Hz). (Found: C,55.4; H,3.9; N,6.2; S,7.2. $C_{21}H_{16}N_2O_8S$ requires C,55.3; H,3.5; N,6.1; S,7.0%).

Example 4(b)

(5RS) Sodium (Z) -6- (5-Acetoxymethylfurfurylidene) penem -3- carboxylate

The penem ester (29) (40 mg) from Example 4(a) was dissolved in a mixture of dioxan (8 ml) and water (2 ml) and was hydrogenated over 5% palladium/charcoal catalyst (60 mg) at S.T.P. for 40 minutes. Further catalyst (40 mg) was added and the hydrogenation continued for 20 minutes. A 1% sodium bicarbonate solution (0.74 ml) was added and the mixture was worked up as for Example 1(b) to give the title sodium salt (30) (13.7 mg) as a yellow solid; $\lambda$ max ($H_2O$) 311 nm (Em 20960); $\delta$ ppm ($D_2O$) 2.14 (3H,s), 5.12 and 5.21 (2H,ABq, J14 Hz), 6.61 (1H,s), 6.65 (1H,d,J4Hz), 6.84 (1H,d,J4Hz), 7.04 (2H,s).

Preparation 5(a)

(3RS, 4SR) 3-[Acetoxy (5-acetoxymethyl-2-furyl) methyl] -4-ethylthiothiocarbonylthio -1- (1-p-nitrobenzyloxycarbonyl -1-triphenylphosphoranylidenemethyl) azetidin -2-one

A stirred, ice bath cooled, suspension of the silver thiolate (27) (460 mg) from Preparation 4(e) in dry dichloromethane (10 ml) was treated with pyridine (125 mg) and ethyl dithiochloroformate (148 mg). The ice bath was removed and the mixture stirred for 45 minutes to give, after work up as for Preparation 2(a), the title phosphorane (31) (370 mg) as a yellow amorphous solid; ν max ($CHCl_3$) 1760, 1745 sh, 1620, 1605 sh.$cm^{-1}$.

Preparation 5(b)

(5RS,6SR) and (5RS,6RS) p-Nitrobenzyl 6-[Acetoxy (5-acetoxymethyl-2-furyl) methyl] -2-ethylthiopenem -3-carboxylate

A solution of the phosphorane (31) (370 mg) from Preparation 5(a) in Xylene (160 ml) was heated under reflux in an atmosphere of argon for 7 hours. Work up as for Preparation 2(b) gave a yellow amorphous solid (93 mg), a 3:1 mixture of the title trans and cis-penems (32) and (33); ν max ($CHCl_3$) 1795, 1745, 1690 $cm^{-1}$; δ ppm ($CDCl_3$) 1.38 (3H,t,J7Hz), 2.07 (6H,s), 2.78-3.18 (2H,m), 4.26 (¾H,dd,J1.8 and 4.9Hz), 4.75 (¼H,dd,J4.0 and 10.3Hz), 5.00 and 5.02 (2H, each s), 5.10-5.60 (2H,m), 5.82 (¼H,d,J4.OHz), 5.90 (¾H,d,J1.8Hz), 6.14-6.55 (3H,m), 7.55 and 7.59 2H, each d, J9Hz), 8.17 and 8.19 (2H, each d, J9Hz).

Example 5(a)

(5RS) p-Nitrobenzyl 6-(5-Acetoxymethylfurfurylidene)-2-ethylthiopenem -3- carboxylate

A solution of DBU (40 mg) in dry dichloromethane (1 ml) was added, dropwise over 1 minute, to a stirred solution ofa mixture of the penem esters (32) and (33) (3:1, 100 mg) from Preparation 5(b) in dry dichloromethane (5 ml) at -40°C. After 10 minutes at -40°C the mixture was worked up as for Example 1(a) to give a mixture of the title (Z) and (E) penems (34) (85 mg) which on crystallisation from chloroform/ether afforded the (Z)-isomer (65 mg) as fine yellow needles, m.p. 204-205°C; λ max (EtOH) 259 (Em 14950) and 323 nm (32490); ν max ($CHCl_3$) 1775, 1740, 1685, 1670 $cm^{-1}$; δ ppm ($CDCl_3$) 1.37 (3H,t,J7Hz), 2.10 (3H,s), 2.60-3.25 (2H,m), 5.08 (2H,s), 5.22 and 5.51 (2H,ABq, J14Hz), 6.43 (1H,s), 6.49 (1H,d,J4Hz), 6.64 (1H,d,J4Hz), 6.86 (1H,s), 7.65 (2H,dJ9Hz), 8.21 (2H,d,J9Hz); (Found: C,53.3; H,4.0; N,5.4; S,12.7; $M^+$, 516.0710. $C_{23}H_{20}N_2O_8S_2$ requires C,53.5; H,3.9; N,5.4; S,12.4% M,516.0660).

Example 5(b)

(5RS) Sodium (z)-6-(5-Acetoxymethylfurfurylidene)-2-ethylthiopenem-3-carboxylate

The penem ester (34) (Z-isomer) (40 mg) from Example 5(a), was dissolved in a mixture of dioxan (8 ml) and water (2 ml) and was hydrogenated over 5% palladium/charcoal catalyst (60 mg) at S.T.P. for 40 minutes. Further catalyst (40 mg) was added and the hydrogenation continued for 20 minutes. A 1% sodium bicarbonate solution (0.65 ml) was added and the mixture was worked up as for Example 1(b) to give the title sodium salt (35) (11.4 mg) as an orange coloured amorphous solid; λ max ($H_2O$) approx. 235 (Em 7430), 315 (23250) and approx. 385 nm (3410); δ ppm ($D_2O$) 1.34 (3H,t,J7Hz), 2.18 (3H,s), 2.88-3.10 (2H,m), 5.17 and 5.27 (2H,ABq, J14Hz), 6.52 (1H,s), 6.69 (1H,d,J3Hz), 6.88 (1H,d,J3Hz), 7.07 (1H,s).

Preparation 6(a)

(3RS,4SR) 3-[Acetoxy (5-hydroxymethyl-2-furyl) methyl] - 1- (1-p-nitrobenzyloxycarbonyl-1-triphenylphosphoranylidenemethyl)-4-tritylthioazetidin-2-one

Aqueous sodium hydroxide (1.0 ml of 0.1N) was added, dropwise over 2 minutes, to a stirred solution of the phosphorane (26) (101 mg) from Preparation 4(d) in a mixture of THF (2 ml) and methanol (0.4 ml) at ice bath temperature. The mixture was stirred for a further 15 minutes at ice bath temperature, treated with 5% citric acid (1 ml) and diluted with ethyl acetate (15 ml). The organic layer was separated and washed with brine (2 ml), saturated $NaHCO_3$ (2 ml) and brine (3 x 2 ml). The dried ($MgSO_4$) organic layer was evaporated and the residue chromatographed on silica gel eluting with ethyl acetate/hexane mixtures to give the title alcohol (36) (65 mg) as an amorphous solid; $\nu$ max ($CHCl_3$) 3600-3100, 1750, 1625, 1605 sh. $cm^{-1}$.

Preparation 6(b)

(3RS,4SR) 3- [Acetoxy (5-formyl-2-furyl) methyl] -1-(1-p-nitrobenzyloxycarbonyl -1-triphenylphosphoranylidenemethyl) -4-tritylthioazetidin -2-one

A solution of the alcohol (36) (50 mg) from Preparation 6(a) in a mixture of dimethyl sulphoxide (0.5 ml) and acetic anhydride (0.5 ml) was kept at room temperature for 6 hours. The mixture was diluted with ethyl acetate (15 ml) and washed with saturated $NaHCO_3$ (2 ml) and brine (3 x 2 ml). The dried ($MgSO_4$) organic layer was evaporated and the residue chromatographed on silica gel eluting with ethyl acetate/hexane mixtures to give the title aldehyde (37) (33 mg) as an amorphous solid; $\nu$ max ($CHCl_3$) 1750, 1680, 1620, 1605 sh.$cm^{-1}$.

Preparation 6(c)

(3RS,4SR) 3- {Acetoxy [5-(2-methoxycarbonylvinyl) -2-furyl ] methyl} -1- (1-p-nitrobenzyloxycarbonyl-1-triphenylphosphoranylidenemethyl)-4-tritylthioazetidin-2-one

A mixture of the aldehyde (37) (32 mg) from Preparation 6(b) and carbomethoxymethylenetriphenylphosphorane (22 mg) in dry dichloromethane (1 ml) was kept at room temperature for 16 hours. The mixture was evaporated and the residue chromatographed on silica gel eluting with ethyl acetate/hexane mixtures to give the title phosphorane (38) (31 mg) as an amorphous solid; ν max ($CHCl_3$) 1750, 1705, 1630 sh., 1615, 1605 $sh.cm^{-1}$.

Preparation 6(d)

(3RS,4SR) Silver 3-{Acetoxy [5-(2-methoxycarbonylvinyl)-2-furyl] methyl} -1-(1-p-nitrobenzyloxycarbonyl-1-triphenylphosphoranylidenemethyl) azetidin-2-one-4-thiolate

The phosphorane (38) (202 mg) from Preparation 6(c) was dissolved in a mixture of methylene chloride (3 ml) and methanol (3 ml) and was treated with pyridine (20 mg) and silver nitrate (1.72 ml of a 0.15M. solution in methanol). After stirring at room temperature for 30 minutes the mixture was evaporated to low volume and filtered. The residue was washed with cold methanol (2 ml) and dry ether (2x2 ml) and dried under vacuum to give the title silver thiolate (39) (169 mg) as a buff coloured amorphous solid.

Preparation 6(e)

(5RS,6SR) p-Nitrobenzyl 6-{Acetoxy [5-(2-methoxycarbonylvinyl)-2-furyl]methyl} penem -3-carboxylate

A stirred, ice bath cooled, suspension of the silver thiolate (39) (169 mg) from Preparation 6(d) in dry dichloromethane was treated with acetic-formic anhydride (0.15 ml), 4-dimethylaminopyridine (21 mg) and triethylamine hydrochloride (263 mg) followed by heating the product as for Preparation 1(f) to give the two title products. The less polar product the (Z)-isomer (40) (5 mg) was obtained as a gum; ν max ($CHCl_3$) 1790, 1740 sh; 1715 $cm^{-1}$; δ ppm ($CDCl_3$) 2.11 (3H,s), 3.77 (3H,s), 4.36 (1H,ddd,J4.1,2.0 and 1.0Hz), 5.28 and 5.42 (2H,ABq, J13.6Hz),5.81 (1H,dJ12.9Hz), 6.07 (1H,d,J2.0Hz), 6.26 (1H,d,J4.1Hz), 6.55 (1H,d,J3.6Hz), 6.69 (1H,d,J12.9Hz), 7.33 (1H,d,J1.0Hz), 7.59 (2H,d,J8.6Hz), 7.60 (1H,d,J3.6Hz), 8.24 (2H,d,J8.6Hz). The more polar product the (E)-isomer (41) (77 mg) was obtained as a solid, m.p. 160-163°C (rods ex ethyl acetate/hexane); λ max (EtOH) 304 nm (Em24000) ν max ($CHCl_3$) 1790, 1740 sh., 1710 $cm^{-1}$; δ ppm ($CDCl_3$) 2.12 (3Hs), 3.79 (3H,s), 4.38 (1H,ddd,J4.3,2.0 and 1.1Hz), 5.29 and 5.43 (2H,ABq,J13.6Hz), 6.09 (1H,d,J2.0Hz), 6.25 (1H,d,J43Hz), 6.28 (1H,d,J15.7Hz), 6.55 (1H,d, J3.4Hz), 6.59 (1H,d,J3.4Hz), 7.34 (1H,d,J1.1Hz), 7.39 (1H,d,J15.7Hz), 7.59 (2H,d,J8.8Hz), 8.24 (2H,d,J8.8Hz); (Found: C,54.6; H,3.7; N,5.1; S,6.1. $C_{24}H_{20}N_2O_{10}S$ requires C,54.6; H,3.8; N,5.3; S,6.1%).

Example 6(a)

(5RS) p-Nitrobenzyl (Z)-6-{5-[(Z)-2-methoxycarbonylvinyl-furfurylidene]} penem -3-carboxylate

A solution of the penem (41) (64 mg) from Preparation 6(e) in dry dichloromethane (3 ml) was treated with DBU (28 mg) at -40$^{o}$C as for Example 1(a) to give the title penem (42) (51 mg) as an orange coloured solid; λ max (EtOH) 249 (Em 19380) and 359 nm (14845) ; ν max ($CHCl_3$) 1775, 1710, 1660, 1630 cm$^{-1}$; δ ppm ($CDCl_3$) 3.82 (eH,s), 5.30-5.47 (2H,ABq, J13.6Hz), 6.46 (1H,d,J15.7Hz), 6.65 (1H,d,J0.9Hz), 6.72 (1H,d,J3.6Hz), 6.79 (1H,d,J3.6Hz), 6.92 (1H,d,J0.9Hz), 7.38 (1H,s), 7.42 (1H,d,J15.7Hz), 7.63 (2H,d,J8.8Hz), 8.25 (2H,d,J8.8Hz).

Example 6(b)

(5RS) Sodium (Z) -6- {5- [(Z) -2- methoxycarbonylvinyl-furfurylidene]} penem -3- carboxylate

The penem ester (43) (40 mg) from Example 6(a) was dissolved in a mixture of dioxan (8 ml) and water (2 ml) and was hydrogenated over 5% palladium/charcoal catalyst (60 mg) at S.T.P. for 45 minutes. A 1% sodium bicarbonate solution (0.72ml) was added and the mixture worked up as for Example 1(b) to give the title sodium salt (43) (12.7 mg) as an orange-yellow amorphous solid; λ max ($H_2O$) 365 (Em 18745) and 252 nm (19960); δ ppm ($D_2O$) 3.80 (3H,s), 6.50 (1H,d,J15.7Hz), 6.71 (1H,s), 6.88 and 6.92 (2H,each d,J 3.7Hz), 7.04 (1H,s), 7.06 (1H,s), 7.46 (1H,d,J15.7Hz).

Preparation 7(a)

1-t-Butyldimethylsilyl-3-[(2-thienyl)hydroxymethyl]-4-tritylthioazetidin-2-one

The title compounds were prepared as described in preparation 1(a) from 4-tritylthioazetidin-2-one (1) (9.18 g, 20 mmol) and thiophene-2-carboxaldehyde (2.46 g, 22 mmol). Chromatography of the crude reaction product gave two fractions. The less polar fraction (3.7 g, 32%) contained an approximately 4:1 mixture of *trans* (isomer A) and *cis* (isomer B) isomers of the title azetidinones (44) as an amorphous solid. $\nu_{max.}$ (DCM) 3650-3350, 1745 $cm^{-1}$. $\delta$ ($CDCl_3$) (*inter alia* for *trans* isomer A) 0.21 (3H, s), 0.24 (3H, s), 0.93 (9H, s), 1.51 (1H, d, J 6.3 Hz), 3.55 (1H, dd, J 2.0 and 2.2 Hz), 3.85 (1H, dd, J 2.3 and 6.2 Hz), 4.51 (1H, d, J 1.9 Hz); $\delta$ ($CDCl_3$) (*inter alia* for *cis* isomer B) 0.38 (3H, s), 0.39 (3H, s), 1.00 (9H, s), 3.03 (1H, d, J 4.6 Hz), 3.49 (1H, dd, J 1.2 and 4.6 Hz), 4.85 (1H, d, J 4.5 Hz). (Found: $MN^+$ 572). The more polar fraction contained the *trans* azetidinone (44) (isomer C) (4.8 g, 42%) m.p. 127-8° (plates *ex* Ether/Hexane) $\nu_{max.}$ (DCM) 3600-3250, 1740 $cm^{-1}$. $\delta$ ($CDCl_3$) -0.26 (3H, s) 0.05 (3H, s), 0.83 (9H, s), 2.48 (1H, d, J 9.0 Hz), 3.71 (1H, dd, J 1.7 and 6.6 Hz), 4.07 - 4.15 (2H, m), 6.65 - 6.72 (1H, m), 6.86 - 6.93 (1H, m), 7.14 - 7.50 (16H, m).. (Found: C, 69.4; H, 6.4; N, 2.6; S, 11.4. $C_{33}H_{37}NO_2S_2Si$ requires C , 69.4; H, 6.5; N, 2.5; S, 11.2%).

The *trans* azetidinone (44) (isomer C) was contaminated with >3% of a *cis* azetidinone (44) (isomer D) [$\delta$ ($CDCl_3$) (*inter alia*) 4.30 (d, J 3.6 Hz)].

Preparation 7(b)

(3RS,4SR)-3-[Acetoxy(2-thienyl)methyl]-1-t-butyldimethylsilyl-4-tritylthioazetidin-2-one

The title compound (45) was prepared as described in preparation 1(b) from the *trans* azetidinone (44) (Isomer C) (4.8 g, 8.41 mmol) from preparation 7(a). Chromatography of the crude reaction product gave two fractions. The less polar fraction (4.47 g, 87%) contained *trans* acetate (45) (Isomer C), m.p. 143-4° (plates *ex.* MeOH); $\nu_{max.}$ (DCM) 1750 $cm^{-1}$; $\delta$ ($CDCl_3$) (excluding $\underline{Me}_2Si$ signals) 0.80 (9H, s), 1.97 (3H, s), 3.86 (1H, dd, $\underline{J}$ 2, 6 Hz), 3.99 (1H, d, $\underline{J}$ 2 Hz), 5.34 (1H, d, $\underline{J}$ 6 Hz), 6.67 - 7.02 (2H, m), 7.16 - 7.67 (16H, m). The more polar fraction (155 mg, 3%) contained the *cis* acetate (45) (Isomer D); white amorphous solid; $\nu_{max.}$ (DCM) 1745 $cm^{-1}$; $\delta$ ($CDCl_3$) (excluding $\underline{Me}_2Si$ signals) 0.92 (9H, s), 1.66 (3H, s), 3.73 (1H, dd, $\underline{J}$ 6.0 and 6.7 Hz), 4.50 (1H, d, $\underline{J}$ 6.0 Hz), 5.85 (1H, d, $\underline{J}$ 6.7 Hz), 6.89 - 7.65 (18H, m).

Preparation 7(c)

(3RS,4SR) 3-[Acetoxy(2-thienyl)methyl]-4-tritylthioazetidin-2-one

A solution of the acetate (45) (Isomer C) (4.29 g, 7 mmol) from preparation 7(b) in THF (50 ml) under argon at room temperature was treated with potassium fluoride (812 mg, 14 mmol) and 18-crown-6 (370 mg, 1.4 mmol). After 40 minutes the reaction mixture was diluted with ethyl acetate (250 ml) and washed with brine (2 x 50 ml). The dried ($MgSO_4$) organic layer was evaporated and the residue chromatographed on silica gel eluting with ethyl acetate/hexane mixtures to give the title azetidinone (46) (Isomer C) (2.89 g, 83%) m.p. 173-4° (plates ex. ethyl acetate/hexane), $\nu_{max.}$ (DCM) 3380, 1770 $cm^{-1}$; $\delta$ ($CDCl_3$) 2.08 (3H, s), 3.56 - 3.80 (1H, m), 4.36 (1H, bs), 4.39 (1H, d, J 2.5 Hz), 6.41 (1H, d, J 6.5 Hz) 7.03 - 7.65 (18H, m).

Preparation 7(d)

(3RS,4SR) 3-[Acetoxy(2-thienyl)methyl]-1-(1-p-nitrobenzyloxycarbonyl-1-triphenylphosphoranylidenemethyl)-4-tritylthioazetidin-2-one

The azetdinones (46) (Isomer C) (2.50 g, 5.0 mmol) from preparation 7(c) and *p*-nitrobenzyl glyoxylate monohydrate (1.36 g, 6.0 mmol) were heated in refluxing benzene (100 ml) followed by treatement with triethylamine (70 µl) as for preparation 1(d) to give the hydroxyesters (47) as an amorphous solid. This material was treated with 2,6-lutidine (0.9 ml, 7.5 mmol) and thionyl chloride (0.55 ml, 7.5 mmol) in dry THF (70 ml) under argon at 20°C as for preparation 1(d) to give the crude chloroester (48) as an amorphous solid. This material, triphenylphosphine (5.25 g, 20 mmol) and 2,6-lutidine (0.7 ml, 6 mmol) were dissolved in dry dioxan (5 ml) and stirred under argon at room temperature for 4 days. Work-up of the mixture as for preparation 1(d) gave the title phosphorane (49) (Isomer C) (3.4 g, 71%) as a light yellow amorphous solid. $\nu_{max.}$ (DCM) 1740 (br), 1610 $cm^{-1}$.

Preparation 7(e)

(3RS,4SR) Silver 3-[Acetoxy(2-thienyl)methyl]-1-(1-p-nitrobenzyloxycarbonyl-1-triphenylphosphoranylidenemethyl)azetidin-2-one-4-thiolate

The title compound (50) was prepared as described in preparation 1(e) from the phosphorane (44) (310 mg, 0.32 mmol) from preparation 7(d): yield 289 mg, buff solid.

Preparation 7(f)



(5RS,6SR)-*p*-Nitrobenzyl 6-[acetoxy(2-thienyl)methyl]-penem-3-carboxylate

The title compound (51) was prepared as described in preparation 1(f) from the silver thiolate (50) (0.32 mmol) from preparation 7(e): yield 78 mg, 53%; m.p. 125-6° (plates *ex.* ethyl acetate/hexane); $\nu_{max.}$ (DCM) 1795, 1740 (sh), 1720 $cm^{-1}$; $\delta$ ($CDCl_3$) 2.08 (3H, s), 4.32 (1H, dd, *J* 2, 5 Hz), 5.22 and 5.41 (2H, ABq, *J* 13 Hz), 5.91 (1H, d, *J* 2 Hz), 6.48 (1H, d, *J* 5 Hz), 6.87 - 7.37 (4H, m), 7.55 (2H, d, *J* 9 Hz), 8.21 (2H, d, *J* 9 Hz); (Found: $MNH_4^+$ 478).

Example 7(a)



(5RS) *p*-Nitrobenzyl 6-(2-thienyl)methylenepenem-3-carboxylate

A mixture of *Z* and *E* isomers of the title compound (52) were prepared as described in Example 1(a) from the penem ester (51) (918 mg, 2 mmol) from preparation 7(f). Chromatography of the crude reaction product on silica eluting with dichloromethane/ethyl acetate mixtures gave two fractions. The less polar fraction contained the *E* isomer (52) (40 mg, 5%) m.p. 199-200°C (yellow needles *ex.* dichloromethane/hexane); $\nu_{max.}$ ($CHCl_3$) 1770, 1720, 1665 $cm^{-1}$; δ ($CDCl_3$), 5.31 and 5.46 (2H, ABq, *J* 13.6 Hz), 6.45 (1H, d, *J* 0.6 Hz), 6.77 (1H, s), 7.13 (1H, dd, *J* 3.8, 5.0 Hz) 7.42 (1H, s), 7.56 (1H, d, *J* 5.1 Hz), 7.63 (1H, d, *J* 8.9 Hz), 7.74 (1H, d, *J* 3.5 Hz) 8.25 (1H, d, *J* 8.7 Hz). The more polar fraction (667 mg, 83%) contained the *Z* isomer (52), m.p. 190-2 (yellow needles *ex.* dichloromethane); $\nu_{max.}$ (DCM) 1780, 1720, 1665 $cm^{-1}$; δ ($CDCl_3$) 6.48 (1H, s), 7. 7.05 - 7.45 (5H, m), 7.60 (2H, d, *J* 8 Hz), 8.23 (2H, d, *J* 8 Hz); (Found: $M^+$ 400.0190. $C_{18}H_{12}N_2O_5S_2$ requires M 400.0188).

Example 7(b)

(5RS) Sodium (Z)-6-(2-thienyl)methylenepenem-3-carboxylate

The title compound (53) was prepared as described in Example 1(b) from the Z penem (52) (40 mg, 0.1 mmol) from Example 7(a). After chromatography on Biogel P2 the appropriate fractions were freeze-dried to give the sodium salt (53) (12.6 mg, 46%) as a yellow solid; $\lambda_{max.}$ ($H_2O$) 308 nm ($\epsilon_m$ 17,600); $\delta$ ($D_2O$) 6.60 (1H, s), 7.06 (1H, s), 7.24 (1H, dd, *J* 3.8, 4.9 Hz), 7.46 (1H,d, *J* 3.3 Hz), 7.50 (1H, s), 7.77 (1H, d, *J* 5.1 Hz).

Example 7(c)



(5RS) Sodium (*E*)-6-(2-thienyl)methylenepenem-3-carboxylate

The title compound (54) was prepared as described in Example 1(b) from the *E* penem (52) (35 mg, 0.088 mmol), from Example 7(a). After chromatography on Biogel P2 the appropriate fractions were freeze-dried to give the sodium salt (54) (10 mg, 41%) as a yellow solid; $\lambda_{max.}$ ($H_2O$) 317 nm ($\epsilon_m$ 11,570); $\delta$ ($D_2O$) 6.47 (1H, s), 7.04 (1H, s), 7.13 (1H, s), 7.18 (1H, dd, *J* 3.7, 5.0 Hz), 7.67 (1H, d, *J* 3.4 Hz), 7.70 (1H, d, *J* 5.0 Hz).

Preparation 8(a)

1-*t*-Butyldimethylsilyl-3-[(3-thienyl)hydroxymethyl]-4-tritylthioazetidin-2-one

The title compounds (55) were prepared as described in preparation 1(a) from 4-tritylthioazetidin-2-one (1) (459 mg, 1 mmol) and thiophen-3-carboxaldehyde (123 mg, 1.1 mmol). Chromatography of the crude reaction product gave three fractions. The least polar fraction (28 mg, 5%) contained a *cis* isomer (Isomer A) of the title azetidinone (55); m.p. 201-2° (prisms *ex.* ethyl acetate/hexane $\nu_{max.}$ (DCM) 3600 - 3400, 1750 $cm^{-1}$; $\delta$ ($CDCl_3$) 0.38 (3H, s), 0.39 (3H, s), 0.99 (9H, s), 2.95 (1H, d, *J* 4.4 Hz, *exch.* with $D_2O$), 3.41 (1H, dd, *J* 1.1, 4.6 Hz), 3.42 (1H, bd, *J* 4.6 Hz, collapses to bs on $D_2O$ exchange), 4.85 (1H, d, *J* 4.5 Hz), 6.92 - 7.05 (1H, m), 7.12 - 7.19 (2H, m) 7.27 - 7.38 (9H, m), 7.46 - 7.55 (6H, m); (Found: $[M + Et_2NH_2]^+$ 645) The second fraction (130 mg, 28%) contained a *trans* isomer (Isomer B) of the title azetidinone (55); m.p. 173-5° (needles *ex.* ethyl acetate/hexane); $\nu_{max.}$ (DCM) 3600 - 3400, 1745 $cm^{-1}$; $\delta$ ($CDCl_3$) 0.20 (3H, s), 0.23 (3H, s), 0.93 (9H, s), 1.38 (1H, d, *J* 6.0 Hz), 3.50 (1H, t, *J* 2.1 Hz), 3.79 (1H, dd, *J* 2.3, 6.0 Hz, collapses to d, *J* 2.3 Hz on irradiation at $\delta$ 1.38), 4.45 (1H, d, *J* 2.0 Hz), 7.02 (1H, dd, *J* 1.3, 4.9 Hz), 7.15 - 7.36 (11H, m), 7.40 - 7.53 (6H, m). The more polar fraction (268 mg, 47%) contained a *trans* isomer (Isomer C) of the title azetidinone (55); m.p. 101-2° (rods *ex.* ethyl acetate/hexane); $\nu_{max.}$ (DCM) 3600 - 3400, 1740 $cm^{-1}$; $\delta$ ($CDCl_3$) 0.08 (3H, s), 0.09 (3H, s), 0.88 (9H, s), 2.55 (1H, d, *J* 9 Hz, exch. with $D_2O$), 3.80 (1H, dd, *J* 1.8, 6.5 Hz), 4.02 (1H, d, *J* 1.7 Hz), 4.15 (1H, dd, *J* 6.5, 8.9 Hz, collapses to d, *J* 6.5 Hz on $D_2O$ exchange), 6.81 (1H, dd, *J* 1.2, 5.0 Hz), 6.88 (1H, dd, *J* 0.7, 2.4 Hz), 7.28 - 7.45 (10H, m), 7.47 - 7.54 (6H, m); (Found: $[M+ Et_2NH_2]^+$ 645.

Preparation 8(b) 

(3RS,4SR) 3-[Hydroxy(3-thienyl)methyl]-4-tritylthioazetidin-2-one

A solution of alcohol (55) (Isomer C) (4.1 g, 7.18 mmol) from preparation 8(a) in methanol (40 ml) at -20°C under argon was treated with a solution of anhydrous potassium fluoride (500 mg, 8.62 mmol) in methanol (10 ml). After one and a half hours the reaction mixture was diluted with dichloromethane (250 ml) and washed with brine (2 x 50 ml). The dried ($MgSO_4$) organic layer was evaporated and the residue triturated with dichloromethane and then filtered to give the title azetidinone (56) (Isomer C) (2.54 g, 77%). The filtrate was evaporated and chromatographed on silica eluting with ethyl acetate/hexane mixtures to give a further amount (0.27 g, 8%) of the title azetidinone (56) (Isomer C); m.p. 199° (prisms ex. ethyl acetate); $\nu_{max.}$ ($CHCl_3$) 3600 - 3400, 3380, 1760 $cm^{-1}$. $\delta$ ($CDCl_3$), 2.72 (1H, bs, exch. with $D_2O$), 3.56 (1H, t, J 3.0 Hz), 4.20 (1H, bs), 4.49 (1H, d , J 2.8 Hz), 5.27 (1H, bd. J 3.2 Hz, sharpens to d, J 3.2 Hz on $D_2O$ exchange), 7.05 - 7.52 (18H, m).

Preparation 8(c)



(3RS,4SR) 3-[Acetoxy (3-thienyl)methyl]-4-tritylthioazetidin-2-one

A solution of the alcohol (56) (Isomer C) (2.7 g, 5.9 mmol) from preparation 8(b) in dichloromethane (100 ml) at room temperature under argon was sequentially treated with triethylamine (0.99 ml, 7.08 mmol), 4-dimethylaminopyridine (86 mg, 0.71 mmol) and acetic anhydride (0.67 ml, 7.08 mmol). After 30 minutes the reaction mixture was diluted with dichloromethane (100 ml) and washed with 1N hydrochloric acid solution (20 ml), saturated $NaHCO_3$ (20 ml) and brine (50 ml). The dried ($MgSO_4$) organic layer was evaporated and the residue chromatographed on silica eluting with ethyl acetate/hexane mixtures to give, after trituration with ether, the title azetidinone (57) (Isomer C) (2.69 g, 91%) as a solid, m.p. 168-9° (prisms ex. ethyl acetate/hexane); $\nu_{max.}$ (DCM) 3380, 1775, 1745 $cm^{-1}$; δ ($CDCl_3$) 2.05 (3H, s), 3.61 (1H, dd, *J* 2, 6 Hz), 4.29 (1H, bs), 4.35 (1H, d, *J* 2 Hz), 6.23 (1H, d, *J* 6 Hz), 7.05 - 7.55 (18H, m).

Preparation 8(d)



(3RS,4SR) 3-[Acetoxy-(3-thienyl)methyl]-1-(1-p-nitrobenzyloxycarbonyl-1-triphenylphosphoranylidenemethyl)-4-tritylthioazetidin-2-one

The title compound (60) (Isomer C) was prepared from the azetidinone (57) (Isomer C) (2.095 g, 4.2 mmol) from preparation 8(c) *via* the hydroxy ester (58) and the chloro ester (59) as described in preparation 7(d); yield 2.91 g, 73%; light yellow amorphous solid; $\nu_{max.}$ (DCM) 1750, 1610 $cm^{-1}$.

Preparation 8(e)



(3RS,4SR) Silver 3-[Acetoxy (3-thienyl)methyl]-1-(1-*p*-nitrobenzyloxycarbonyl-1-triphenylphosphoranylidenemethyl)azetidin-2-one-4-thiolate

The title compound (61) (Isomer C) was prepared from the phosphorane (60) (Isomer C) (2.9 g, 3.05 mmol) from preparation 8(d) as described preparation 1(e); yield 2.49 g, 100%, white solid.

Preparation 8(f)



(5RS,6SR) *p*-Nitrobenzyl 6-[Acetoxy-(3-thienyl)methyl]-penem-3-carboxylate

The title compound (62) (Isomer C) was prepared from the silver thiolate (61) (Isomer C) (2.49 g, 3.05 mmol) from preparation 8(e) as described in preparation 1(f); yield 983 mg, 70%, m.p. 140-2° (plates *ex.* ethyl acetate/hexane); $\nu_{max.}$ (DCM) 1795, 1740, 1720 $cm^{-1}$; $\delta$ ($CDCl_3$) 2.08 (3H, s), 4.32 (1H, dd, *J* 2, 5 Hz), 5.22 and 5.41 (2H, ABq, *J* 13 Hz), 5.91 (1H, d, *J* 2 Hz), 6.48 (1H, d, *J* 5 Hz), 6.87 - 7.37 (4H, m), 7.55 (2H, d, *J* 9 Hz), 8.21 (2H, d, *J* 9 Hz); (Found: $MNH_4^{\oplus}$ 478).

Example 8(a)

(5RS) *p*-Nitrobenzyl 6-(3-thienyl)methylenepenem-3-carboxylate

A mixture of *Z* and *E* isomers of the title compound (63) were prepared as described in Example 1(a) from the penem ester (62) (820 mg, 2 mmol) from preparation 8(f). Chromatography of the crude reaction product on silica eluting with dichloromethane/ethyl acetate mixtures gave two fractions. The less polar fraction (130 mg, 16%) contained the *E* isomer (63) m.p. 156-70° (yellow needles *ex.* dichloromethane/hexane); $\lambda_{max.}$ (EtOH) 306 nm ($\varepsilon_m$ 24,220); $\nu_{max.}$ (DCM) 1770, 1720, 1665 $cm^{-1}$; $\delta$ ($CDCl_3$) 5.30 and 5.46 (2H, ABq, *J* 13.6 Hz), 6.44 (1H, d, *J* 0.59 Hz), 6.69 (1H, s), 7.37 (1H, dd, *J* 2.9, 5.0 Hz), 7.43 (1H, s), 7.63 (2H, d, *J* 8.8 Hz), 7.83 (1H, dd, *J* 1.1, 5.1 Hz), 7.97 (1H, dd, *J* 1.0, 2.7 Hz), 8.25 (2H, 3, *J* 8.8 Hz). The more polar fraction (645 mg, 81%) contained the *Z* isomer (63), m.p. 182-3° (yellow needles *ex* dichloromethane); $\lambda_{max.}$ (EtOH) 295 nm ($\varepsilon_m$ 24,240); $\nu_{max.}$ (DCM) 1785, 1720, 1675 $cm^{-1}$; $\delta$ ($CDCl_3$) 5.30 and 5.46 (2H, ABq, *J* 13.6 Hz), 6.57 (1H, d, *J* 1.0 Hz), 7.04 (1H, dd, *J* 1.2, 5.1 Hz), 7.22 (1H, bs), 7.36 (1H, s), 7.45 (1H, dd, *J* 3.2, 5.1 Hz), 7.53 (1H, dd, *J* 1.2, 3.0 Hz), 7.63 (2H, d, *J* 8.9 Hz), 8.25 (2H, d, *J* 8.8 Hz). (Found: $M^+$ 400.0190. $C_{18}H_{12}N_2O_5S$ requires M 400.0188).

Example 8(b)

(5RS) Sodium (Z)-6-(3-thienyl)methylenepenem-3-carboxylate

The title compound (64) was prepared as described in Example 1(b) from the Z penem (63) (75 mg, 0.19 mmol) from Example 7(a). After chromatography on Biogel P2 the appropriate fractions were combined and freeze-dried to give the sodium salt (64) (30 mg, 55%) as a yellow solid; $\lambda_{max.}$ ($H_2O$) 296 nm ($\varepsilon_m$ 22,510); $\delta$ ($D_2O$) 6.69 (1H, s), 7.04 (1H, s), 7.18 (1H, d, *J* 5.0 Hz), 7.27 (1H, bs), 7.57 (1H, dd, *J* 3.0, 5.0 Hz), 7.76 (1H, bs).

Example 8(c)

(5RS) Sodium *E*-6-(3-thienyl)methylenepenem-3-carboxylate

The title compound (65) was prepared as described in Example 1(b) from the *E* penem (63) (40 mg, 0.1 mmol) from Example 8(a). After chromatography on Biogel P2 the appropriate fractions were freeze-dried to give the sodium salt (65) (15 mg, 52% as a yellow solid; $\lambda_{max.}$ ($H_2O$) 298 nm ($\varepsilon_m$ 14,980); $\delta$ ($D_2O$) 6.47 (1H, s), 6.90 (1H, s), 7.13 (1H, s), 7.51 (1H, dd, *J* 3.0, 5.0 Hz), 7.80 (1H, d, *J* 5.0 Hz), 8.01 (1H, bs).

Preparation 9(a) 

(3RS,4SR) and (3RS,4RS) 3-[Acetoxy-(2-furyl)methyl]-1-*t*-butyldimethylsilyl-4-tritylthioazetidin-2-one

The mixture of *trans* (Isomer A) and *cis* (Isomer B) azetidinones (2) (9.33 g) from Preparation 1(a) was treated as for Preparation 1(b) to give the title compounds (66)(4.09 g), a 3:2 mixture of *trans* and *cis* isomers, as an amorphous solid, $\nu_{max.}$ ($CHCl_3$) 1745 $cm^{-1}$; δ ppm ($CDCl_3$) 0.90 and 1.00 (9H, each s, ratio 3:2), 1.84 and 2.00 (3H, each s, ratio 3:2), 3.44 (0.4H, dd, 4½ and 1 Hz), 3.69 (0.6H, dd, J 2 and 2 Hz), 4.09 (0.6H, d, J 2 Hz), 4.69 (0.4H, d, J 1 Hz), 4.79 (0.4H, d, J 4½ Hz), 5.08 (0.6 H, d, J 2 Hz), 6.15 - 6.45 (2H, m), 7.10 - 7.60 (16H, m), both $SiMe_2$ signals were obscured by the TMS signal.

Preparation 9(b)



(3RS,4SR) and (3RS,4RS) 3-[Acetoxy-(2-furyl)methyl]-4-tritylthioazetidin-2-one

The mixture of *trans* and *cis* azetidinones (66) (6.1 g) from Preparation 9(a) was treated as for Preparation 1(c) to give the title compounds (67) (3.41 g), a 3:2 mixture of *trans* and *cis* isomers, as an amorphous solid, $\nu_{max.}$ ($CHCl_3$) 3380, 1765 $cm^{-1}$; $\delta$ ppm ($CDCl_3$) 2.03 (3H, s), 3.73 (0.4H, dd, J 9 and 3 Hz), 4.04 (0.6H, dd, J 6 and 6 Hz), 4.21 (d, J 3 Hz) and 4.28 br (s) together 1H, 4.51 (d, J 6 Hz) and 4.72 br (s) together 1H, 6.12 (d, J 9 Hz) and 6.29 (d, J 6 Hz) together 1H, 6.35 - 6.65 (2H, m), 7.10 - 7.50 (16H, m).

Preparation 9(c)



(3RS,4SR) and (3RS,4RS) 3-[Acetoxy-(2-furyl)methyl]-1-(1-*p*-nitrobenzyloxycarbonyl-1-triphenylphosphoranylidenemethyl)-4-tritylthioazetidin-2-one

The mixture of *trans* and *cis* azetidinones (67) (3.95 g) from Preparation 9(b) was treated as for Preparation 1(d) to give a mixture of the title compounds (68) (4,77 g) as an amorphous solid, $\nu_{max.}$ ($CHCl_3$) 1750, 1620 sh, 1615, 1605 sh $cm^{-1}$.

Preparation 9(d) 

(3RS,4SR) and (3RS,4RS) Silver 3-[Acetoxy-(2-furyl)-methyl]-1-(1-*p*-nitrobenzyloxycarbonyl-1-triphenylphosphoranylidenemethyl)azetidin-2-one-4-thiolate

The mixture of phosphoranes (68) (4.77 g) from Preparation 9(c) was treated with silver nitrate as in Preparation 1(e) to give the title silver salts (69) (1.18 g) as a buff coloured amorphous solid, $\nu_{max.}$ (Nujol) 1750, 1600 $cm^{-1}$. Evaporation of the filtrate and trituration of the residue with dry ether gave a further crop of the silver salt contaminated with silver nitrate (3.32 g).

Preparation 9(e)

(5RS,6SR) and (5RS,6RS) *p*-Nitrobenzyl 6-[Acetoxy-(2-furyl)methyl]penem-3-carboxylate

The mixture of silver salts (69) (4.50 g, containing silver nitrate as contaminant from Preparation 9(d) was treated as for Preparation 1(f) to give the title penems (70) (1.85 g), a 3:1 mixture of *trans* and *cis* isomers, as an amorphous solid, $\nu_{max.}$ ($CHCl_3$) 1800, 1740 sh., 1720 $cm^{-1}$; $\delta$ ppm ($CDCl_3$) 2.10 (3H, s), 4.41 (0.75H, dd, J 6 and 2 Hz), 4.64 (0.25H, dd, J 11 and 4 Hz), 5.20 and 5.40 (2H, ABq, J 14 Hz), 5.64 (d, J 2 Hz) and 5.71 (d, J 4 Hz) together 1H, 6.25 - 6.60 (3H, m), 7.25 - 7.45 (2H, m), 7.55 (2H, d, J 9 Hz), 8.19 (2H, d, J 9 Hz).

Example 9(a)

(5RS) *p*-Nitrobenzyl (*E*)-6-Furfurylidenepenem-3-carboxylate and (5RS) *p*-Nitrobenzyl (*Z*)-6-Furfurylidenepenem-3-carboxylate

The mixture of *trans* and *cis*-penems (70) from Preparation 9(e) (1.70 g) was treated as for Example 1(a) to give two products after chromatography on silica gel eluting with dichloromethane/ethyl acetate mixtures. The less polar product, the (*E*)-penem (71) (631 mg) was obtained as an orange microcrystalline solid, m.p. 156-159°C (*ex.* ethyl acetate/hexane); $\lambda_{max.}$ (EtOH) 325 ($\varepsilon_m$ 23,142) and 262 nm (14,775); $\nu_{max.}$ ($CHCl_3$) 1770, 1715, 1665 $cm^{-1}$; $\delta$ ppm ($CDCl_3$) 5.24 and 5.45 (2H, ABq, J 14 Hz), 6.41 (1H, s), 6.53 (2H, s), 7.39 (1H, s), 7.45 - 7.70 (4H, m), 8.22 (2H, d, J 8 Hz). (Found: C, 56.1; H, 3.1; N, 7.2; S, 8.2; $M^+$, 384.0418. $C_{18}H_{12}N_2O_6S$ requires C, 56.3; H, 3.1; N, 7.3; S, 8.3%; M, 384.0416). The more polar isomer, the (*Z*)-penem (10) (528 mg) was obtained as an orange yellow solid.

Example 9(b)

(5RS) Sodium (*E*)-6-Furfurylidenepenem-3-carboxylate

The (*E*)-penem (71) (100 mg) from Example 9(a) was treated as for Example 1(b) to give, after chromatography on Biogel P2 eluting with water and freeze-drying, the title sodium salt (72) (27.4 mg) as a yellow amorphous solid, $\lambda_{max.}$ ($H_2O$) 318 nm ($\varepsilon_m$ 13335); $\nu_{max.}$ (KBr) 3700 - 2700, 1750, 1665, 1610, 1560 $cm^{-1}$; $\delta$ ppm ($D_2O$) 6.45 (1H, s), 6.63 (1H, dd, J 3.3 and 1.7 Hz), 6.73 (1H, s), 7.10 - 7.15 (2H, m), 7.70 br (1H, s).

Preparation 10(a)

(5RS,6SR) *p*-Nitrobenzyl 6-[Acetoxy-(2-furyl)methyl]-2-(2-*p*-nitrobenzyloxycarbonylethylthio)penem-3-carboxylate

The penem sulphoxide (17) (80 mg) from Preparation 3(a) was treated with *p*-nitrobenzyl 2-mercaptopropionate (74 mg) and diisopropylethylamine (20 mg) under the conditions described in Preparation 3(b) to give the title penem (73) (62 mg) as a pale yellow amorphous solid, $\lambda_{max.}$ (EtOH) 262 ($\varepsilon_m$, 20,267) and 335 nm (8181); $\nu_{max.}$ ($CHCL_3$) 1795, 1740, 1690 $cm^{-1}$; $\delta$ ppm ($CDCl_3$) 2.03 (3H, s), 2.65 - 3.40 (4H, m), 4.26 (1H, dd, J 5 and 2 Hz), 5.19 and 5.42 (ABq, J 14 Hz) and 5.21 (s) together 4H, 5.90 (1H, d, J 2 Hz), 6.22 (1H, d, J 5 Hz), 6.30 - 6.45 (2H, m), 7.35 - 7.65 (5H, m), 8.18 (4H, d, J 8 Hz).

Example 10(a)

(5RS) *p*-Nitrobenzyl (Z)-6-Furfurylidene-2-(2-*p*-nitrobenzyloxycarbonylethylthio)penem-3-carboxylate

The penem (73) (55 mg) from Preparation 10(a) was treated as in Example 1(a) to give the title penem (74) (42 mg) as a yellow amorphous solid, $\lambda_{max.}$ (EtOH) 262 ($\varepsilon_m$ 13,939) and 329 nm (17,480); $\nu_{max.}$ ($CHCl_3$) 1770, 1735, 1685 sh., 1670 $cm^{-1}$; $\delta$ ppm ($CDCl_3$) 2.85 (2H, t, J 7.2 Hz), 3.14 - 3.36 (2H, m), 5.24 (2H, s), 5.25 and 5.51 (2H, ABq, J 13.8 Hz), 6.48 (1H, d, J 1.0 Hz), 6.57 (1H, dd, J 3.5 and 1.9 Hz), 6.75 (1H, d, J 3.5 Hz), 6.94 (1H, slightly broadened s), 7.51 (2H, d, J 8.7 Hz), 7.60 (1H, d, J 1.6 Hz), 7.67 (2H, d, J 8.7 Hz), 8.22 and 8.23 (4H, each d, J 8.7 Hz).

Example 10(b)

(5RS) 2-(2-Carboxyethylthio)-(Z)-6-furfurylidenepenem-3-carboxylate Disodium Salt

The penem (74) (40 mg) from Example 10(a) was hydrogenated as for Example 1(b). Treatment of the reaction mixture with 1% sodium bicarbonate solution (1.08 ml) followed by work-up as for Example 1(b) gave, after chromatography on Biogel P2 eluting with water and freeze-drying, the title disodium salt (75) (13.1 mg) as an amorphous red-brown solid, $\lambda_{max.}$ ($H_2O$) 312 nm; δ ppm ($D_2O$) 2.56 - 2.65 (2H, m), 2.98 - 3.25 (2H, m) 6.56 (1H, d, J 0.9 Hz), 6.65 (1H, dd, J 3.5 and 1.8 Hz), 6.91 (1H, d, J 3.5 Hz), 7.09 br (1H, s), 7.76 (1H, d, J 1.8 Hz).

Preparation 11(a)

3-[(5-Bromo-2-furyl)hydroxymethyl]-1-*t*-butyldimethylsilyl-4-tritylthioazetidin-2-one

A solution of *n*-butyl lithium (1.6 M in hexane, 0.75 ml) was added to a stirred solution of diisopropylamine (0.17 ml) in dry THF (8 ml) at -30°C under dry argon. After 10 minutes at -30°C the stirred mixture was cooled to -76°C and treated with a solution of 1-*t*-butyldimethylsilyl-4-tritylthioazetidin-2-one (1) (459 mg) in dry THF (4 ml). After 15 minutes at -76°C the stirred mixture was treated with a solution of 5-bromofurfuraldehyde (192 mg) in dry THF (1 ml). The mixture was stirred at -76°C for a further 10 minutes, treated with saturated ammonium chloride solution (5 ml), and worked up as for Preparation 1(a) to give two fractions. The less polar fraction (168 mg) contained an approximately 1:1 mixture of *trans* (Isomer A) and *cis* (Isomer B) isomers of the title azetidinone (76), $\nu_{max.}$ ($CHCl_3$) 3600 - 3100, 1740 $cm^{-1}$; δ ppm ($CDCl_3$) 0.90 and 0.94 (9H, each s), 1.3 - 1.9 (1H, broad signal, exch. $D_2O$), 3.35 - 3.65 (2H, m, sharpens on exch. $D_2O$), 4.44 (0.5H, d, J 2 Hz), 4.75 (0.5H), d, J 5 Hz), 6.06 - 6.34 (2H, m), 7.10 - 7.60 (15H, m), the $Me_2Si$ signals were obscured by the TMS signal. The more polar fraction (326 mg) contained a single *trans*-isomer (Isomer C) of the title azetidinone (76), $\nu_{max.}$ ($CHCl_3$) 3600 - 3100, 1730 $cm^{-1}$; δ ppm ($CDCl_3$) 0.82 (9H, s), 2.45 - 2.66 br (1H, m, exch. $D_2O$), 3.66 (1H, dd, J 7 and 2 Hz), 3.75 - 4.00 (1H, m, collapses to 3.85, d, J 7 Hz on exch. $D_2O$), 4.10 (1H, d, J 2 Hz), 5.99 (1H, d, J 3 Hz), 6.13 (1H, d, J 3 Hz), 7.15 - 7.55 (15H, m), the $Me_2Si$ signals were obscured by the TMS signal.

Prepaation 11(b)

(3RS,4SR) 3-[Acetoxy(5-bromo-2-furyl)methyl]-1-*t*-butyldimethylsilyl-4-tritylthioazetidin-2-one

The *trans*-azetidinone (76) (Isomer C) (2.19 g) from Preparation 11(a) was acetylated as for Preparation 1(b) to give the title compound (77) (2.08 g) as an amorphous solid, $\nu_{max.}$ ($CHCl_3$) 1740 $cm^{-1}$; $\delta$ ppm ($CDCl_3$) 0.83 (9H, s), 1.93 (3H, s), 3.84 (1H, dd, J 6 and 2 Hz), 3.92 (1H, d, J 2 Hz), 5.12 (1H, d, J 6 Hz), 6.05 (1H, d, J 3 Hz), 6.14 (1H, d, J 3 Hz), 7.00 - 7.70 (15H, m), the $Me_2Si$ signals were obscured by the TMS signal.

Preparation 11(c)

(3RS,4SR) 3-[Acetoxy(5-bromo-2-furyl)methyl]-4-tritylthioazetidin-2-one

The acetate (77) (1.80 g) from Preparation 11(b) was treated with potassium fluoride and 18-crown-6 as for Preparation 4(c) to give the title azetidinone (78) (1.49 g) as an amorphous solid, $\nu_{max.}$ ($CHCl_3$) 3380, 1770 $cm^{-1}$; $\delta$ ppm ($CDCl_3$) 2.07 (3H, s), 3.60 br (1H, dd, J 6 and 3 Hz), 4.20 br (1H, s), 4.56 (1H, d, J 3 Hz), 6.06 (1H, d, J 6 Hz), 6.26 and 6.37 (2H, each d, J 3 Hz), 7.10 - 7.60 (15H, m).

Preparation 11(d)

(3RS,4SR) 3-[Acetoxy(5-bromo-2-furyl)methyl]-1-(1-*p*-nitrobenzyloxycarbonyl-1-triphenylphosphoranylidenemethyl)-4-tritylthioazetidin-2-one

The azetdinone (78) (1.49 g) from Preparation 11(c) was treated as for Preparation 1(d) to give the title phosphorane (79) (1.58 g) as an amorphous solid, $\nu_{max.}$ ($CHCl_3$) 1750, 1620, 1610 sh. $cm^{-1}$.

Preparation 11(e)

(5RS,6SR) *p*-Nitrobenzyl 6-[Acetoxy(5-bromo-2-furyl)-methyl]penem-3-carboxylate

The phosphorane (79) (200 mg) from Preparation 11(d) was dissolved in a mixture of methanol (2 ml) and dichloromethane (2 ml) and treated with pyridine (20 mg) and silver nitrate (1.71 ml of a 0.15M solution in methanol). After stirring at room temperature for 30 minutes the mixture was evaporated to low volume and diluted with dry ether. The precipitated solid was filtered, washed with dry ether (2 x 5 ml) and dried under vacuum to give crude silver thiolate (80) (184 mg) as an amorphous solid.

The crude silver thiolate (80) was treated as for Preparation 1(f) to give the title penem (81) (78 mg) as a pale yellow amorphous solid, $\lambda_{max.}$ (EtOH) 261 ($\varepsilon_m$ 5398) and 312 nm (3246); $\nu_{max.}$ ($CHCl_3$) 1795, 1740 sh, 1720 $cm^{-1}$; $\delta$ ppm ($CDCl_3$) 2.06 (3H, s), 4.30 (1H, dd, J 2 and 5 Hz), 5.20 and 5.41 (2H, ABq, J 14 Hz), 5.99 (1H, d, J 2 Hz), 6.14 (1H, d, J 5 Hz), 6.26 (1H, d, J 4 Hz), 6.40 (1H, d, J 4 Hz), 7.29 (1H, s), 7.65 (2H, d, J 9 Hz), 8.20 (2H, d, J 9 Hz).

Example 11(a)

(5RS) *p*-Nitrobenzyl (Z)-6-(5-Bromofurfurylidene)penem-3-carboxylate

The penem (81) (69 mg) from Preparation 11(e) was treated as for Example 1(a) to give the title penem (82) (52 mg) as a photolabile yellow solid, $\lambda_{max.}$ (EtOH) 262 ($\varepsilon_m$ 10,635) and 320 nm (23,242); $\nu_{max.}$ ($CHCl_3$) 1775, 1710, 1660 $cm^{-1}$; $\delta$ ppm ($CDCl_3$) 5.28 and 5.46 (2H, ABq, J 13.6 Hz), 6.49 (1H, d, J 3.5 Hz), 6.58 (1H, d, J 1.0 Hz), 6.68 (1H, d, J 3.5 Hz), 6.85 (1H, d, J 1.0 Hz), 7.38 (1H, s), 7.62 (2H, d, J 8.7 Hz), 8.25 (2H, d, J 8.7 Hz). (Found: $M^+$, 461.9522. $C_{18}H_{11}N_2O_6SBr$ requires M, 461.9522).

Example 11(b)

(5RS) Sodium (Z)-6-(5-Bromofurfurylidene)penem-3-carboxylate

The penem (82) (48 mg) from Example 11(a) was hydrogenated as for Example 1(b) to give the title sodium salt (83) (6.7 mg) as a yellow brown amorphous solid, $\lambda_{max.}$ ($H_2O$) 310 nm; $\delta$ ppm ($D_2O$) 6.64 (d, J 3.6 Hz) and 6.66 (d, J 1.0 Hz) together 2H, 6.87 (1H, d, J 3.6 Hz), 7.02 (1H, slightly broadened s), 7.09 (1H, s).

Preparation 12(a)

(3RS,4SR) 1-*t*-Butyldimethylsilyl-3-(3-furoyl)-4-tritylthioazetidin-2-one

A solution of *n*-butyl lithium (1.6M in hexane, 0.75 ml) was added to a stirred solution of diisopropylamine (0.17 ml) in dry THF (8 ml) at -30°C under dry argon. After stirring at -30°C for 10 minutes the mixture was cooled to -76°C and treated with a solution of 1-*t*-butyldimethylsilyl-4-tritylthioazetidin-2-one (459 mg) in dry THF (4 ml). After 15 minutes at -76°C the stirred mixture was treated with a solution of ethyl 3-furoate (280 mg) in dry THF (1 ml). The mixture was stirred at -76°C for a further 30 minutes, treated with saturated ammonium chloride (5 ml), and worked up as for Preparation 1(a) to give the title ketone (84) (457 mg) as a solid, m.p. 150 - 151°C (*ex.* hexane), $\nu_{max.}$ ($CHCl_3$) 1745, 1670 $cm^{-1}$; δ ppm ($CDCl_3$) 0.93 (9H, s), 3.84 (1H, d, J 2 Hz) 5.02 (1H, d, J 2 Hz), 6.55 - 6.65 (1H, m), 7.00 - 7.60 (16H, m), 7.75 - 7.85 (1H, m), the $Me_2Si$ signals were observed by the TMS signal. (Found: C, 71.3; H, 6.3; N, 2.5; S, 5.7. $C_{33}H_{35}NO_3SSi$ requires C, 71.6; H, 6.4; N, 2.5; S, 5.8%).

Preparation 12(b)

(3RS,4SR) 1-*t*-Butyldimethylsilyl-3-[(3-furyl)hydroxymethyl]-4-tritylthioazetidin-2-one

Sodium borohydride (27 mg) was added to a stirred, ice-bath cooled, solution of the ketone (84) (100 mg) from Preparation 12(a) in a mixture of dry THF (2 ml) and ethanol (2 ml). The mixture was stirred at room temperature for 3 hours, carefully diluted with brine (5 ml) and 5% citric acid (5 ml), and extracted with ethyl acetate (25 ml). The organic layer was seprated and washed with brine (3 ml), saturated $NaHCO_3$ solution (3 ml), and brine (3 x 3 ml). The dried ($MgSO_4$) organic layer was evaporated and the residue chromatographed on silica gel eluting with ethyl acetate/hexane mixtures to give two isomers of the title *trans*-azetidinone (85). The less polar isomer (Isomer A) (17 mg) was obtained as a solid, m.p. 182-183°C (chunks *ex.* ethyl acetate/hexane), $\nu_{max.}$ ($CHCl_3$) 3600 - 3100, 1740 $cm^{-1}$; $\delta$ ppm ($CDCl_3$) 0.92 (9H, s), 1.41 br (1H, s, exch. $D_2O$), 3.40 (1H, dd, J 2 and 2 Hz), 3.58 - 3.72 (1H, m, sharpens to d, J 2 Hz, on exch. $D_2O$), 4.45 (1H, d, J 2 Hz), 6.40 - 6.50 (1H, m), 7.15 - 7.65 (17H, m), the $Me_2Si$ signals were obscured by the TMS signals. (Found: C, 71.3; H, 6.7; N, 2.6; S, 5.6. $C_{33}H_{37}NO_3SSi$ requires C, 71.3; H, 6.7; N, 2.5; S, 5.8%). The more polar isomer (Isomer B) (60 mg) was obtained as a solid, m.p. 120-121°C (plates *ex.* hexane), $\nu_{max.}$ ($CHCl_3$) 3600 - 3100, 1730 $cm^{-1}$; $\delta$ppm ($CDCl_3$) 0.83 (9H, s), 2.18 br (1H, s, exch. $D_2O$), 3.69 (1H, dd, J 6 and 2 Hz), 3.90 - 4.10 (2H, m, collapses to a d, J 6 Hz, and a d J 2 Hz on exch. $D_2O$), 6.11 - 6.20 (1H, m), 7.04 - 7.13 (1H, m), 7.20 - 7.60 (16H, m), the $Me_2Si$ signals were obscured by the TMS signal. (Found: C, 71.1; H, 6.7; N, 2.4; S, 5.6. $C_{33}H_{37}NO_3SSi$ requires C, 71.3; H, 6.7; N, 2.5; S, 5.8%).

Preparation 12(c)

(3RS,4SR) 3-[Acetoxy(3-furyl)methyl]-1-*t*-butyldimethylsilyl-4-tritylthioazetidin-2-one

The azetidinone (85) (Isomer B) (125 mg) from Preparation 12(b) was treated as for Preparation 1(b) to give the title acetate (86) (108 mg) as a solid, m.p. 152-153°C (chunks *ex.* ethyl acetate/hexane); $\nu_{max.}$ ($CHCl_3$) 1745 $cm^{-1}$; $\delta$ ppm ($CDCl_3$) 0.81 (9H, s), 1.92 (3H, s), 3.81 (dd, J 6 and 2 Hz) and 3.83 (d, J 2 Hz) together 2H, 5.14 (1H, d, J 6 Hz), 6.15 - 6.25 (1H, m), 7.05 - 7.60 (17H, m), the $Me_2Si$ signals were obscured by the TMS signal. (Found: C, 69.8; H, 6.4; N, 2.3; S, 5.1. $C_{35}H_{39}NO_4SSi$ requries C, 70.3; H, 6.6; N, 2.3; S, 5.4%).

Preparation 12(d)

(3RS,4SR) 3-[Acetoxy(3-furyl)methyl]-4-tritylthioazetidin-3-one

The azetidinone (86) (86 mg) from Preparation 12(c) was treated as for Preparation 4(c) to give the title azetidinone (87) (68 mg) as a solid, m.p. 159-160°C (prisms *ex.* ethyl acetate/hexane); $\nu_{max.}$ ($CHCl_3$) 3380, 1770 $cm^{-1}$; δ ppm ($CDCl_3$) 2.05 (3H, s), 3.58 (1H, ddd, J 6, 3 and approximately 1 Hz), 4.36 (1H, d, J 3 Hz), 4.44 (1H, broad signal), 6.12 (1H, d, J 6 Hz), 6.36 - 6.48 (1H, m), 7.15 - 7.60 (17H, m). (Found: C, 72.0; H, 5.3; N, 2.8; S, 6.1. $C_{29}H_{25}NO_4S$ requires C, 72.0; H, 5.2; N, 2.9; S, 6.6%).

Preparation 12(e)

(3RS,4SR) 3-[Acetoxy(3-furyl)methyl]-1-(1-*p*-nitrobenzyloxycarbonyl-1-triphenylphosporanylidenemethyl)-4-tritylthioazetidin-2-one

The azetidinone (87) (966 mg) from Preparation 12(d) was treated with *p*-nitrobenzyl glyoxylate monohydrate and triethylamine followed by thionyl chloride and 2,6-lutidine as for Preparation 1(d) to give the crude chloroester (88). The crude chloroester (88) and triphenylphosphine (2.1 g) were dissolved in dry dioxane (5 ml). The mixture as concentrated to approximately half volume and treated with 2,6-lutidine (0.28 ml). The resulting viscous oil was stirred at 40°C under dry argon for 36 hours. The mixture was diluted with ethyl acetate (30 ml) and washed with 5% citric acid (5 ml), brine (5 ml), saturated $NaHCO_3$ solution (5 ml), and brine (3 x 5 ml). The dried ($MgSO_4$) organic layer was evaporated and chromatographed on silica gel eluting with ethyl acetate/hexane to give the title phosphorane (89) (1.27 g) as an amorphous solid, $\nu_{max.}$ ($CHCl_3$) 1745, 1610 br. $cm^{-1}$.

Preparation 12(f)

(3RS,4SR) Silver 3-[Acetoxy(3-furyl)methyl]-1-(1-*p*-nitrobenzyloxycarbonyl-1-triphenylphosphoranylidenemethyl)azetidin-2-one-4-thiolate

The phosphorane (89) (1.34 g) from Preparation 12(e) was treated as for Preparation 1(e) to give the title silver salt (90) (1.12 g) as an amorphous solid, $\nu_{max.}$ ($CHCl_3$) 1750 $cm^{-1}$.

Preparation 12(g)

(5RS,6SR) *p*-Nitrobenzyl 6-[Acetoxy(3-furyl)methyl]-penem-3-carboxylate

The silver salt (90) (400 mg) from Preparation 12(f) was treated as for Preparation 1(f) to give the title penem (91) (176 mg) as an amorphous solid, $\nu_{max.}$ ($CHCl_3$) 1795, 1720 $cm^{-1}$; $\delta$ ppm ($CDCl_3$) 2.08 (3H, s), 4.24 (1H, ddd, J 6, 2 and approximately 1 Hz), 5.21 and 5.41 (2H, ABq, J 14 Hz), 5.75 (1H, d, J 2 Hz), 6.20 (1H, d, J 6 Hz), 6.39 (1H, br. s), 7.28 (1H, d, J approximately 1 Hz), 7.40 (1H, dd, J 1½ and 1½ Hz), 7.48 (1H, br.s), 7.55 (2H, d, J 9 Hz), 8.21 (2H, d, J 9 Hz). (Found: $M^+$, 444.0622. $C_{20}H_{16}N_2O_8S$ requires M, 444.0627).

Example 12(a)

(5RS) *p*-Nitrobenzyl (*E*)-6-(3-Furylmethylene)penem-3-carboxylate and (5RS) *p*-Nitrobenzyl (*Z*)-6-(3-Furylmethylene)penem-3-carboxylate

The penem (91) (156 mg) from Preparation 12(g) was treated as for Example 1(a) to give two fractions after chromatography on silica gel eluting with dichloromethane/ethyl acetate mixtures. The less polar fraction contained the title (*E*)-penem (92) (19 mg), an amorphous solid, $\lambda_{max.}$ (EtOH) 300 ($\epsilon_m$ 17,250) and 262 nm (16,500); $\nu_{max.}$ ($CHCl_3$) 1770, 1710, 1670 $cm^{-1}$; $\delta$ ppm ($CDCl_3$) 5.30 and 5.46 (2H, ABq, J 13.6 Hz), 6.42 (1H, s), 6.57 (1H, s), 7.42 (1H, s), 7.46 (1H, br. s), 7.62 (2H, d, J 8.8 Hz), 7.89 (1H, br. s), 8.25 (2H, d, J 8.8 Hz), the signal for 1 proton was apparently obscured by the $CHCl_3$ signal. (Found: $M^+$, 384.0418. $C_{18}H_{12}N_2O_5S$ requires M, 384.0416). The more polar fractioncontained the title (*Z*)-penem (93) (97 mg), a solid, m.p. 170-185°C (slow decomposition (fine needles *ex.* ethyl acetate/hexane); $\lambda_{max.}$ (EtOH) 295 nm ($\epsilon_m$ 23,000); $\nu_{max.}$ ($CHCl_3$) 1780, 1720, 1680 $cm^{-1}$; $\delta$ ppm ($CDCl_3$) 5.29 and 5.46 (2H, ABq, J 13.6 Hz), 6.34 - 6.35 (1H, m), 6.53 (1H, d, J 1.1 Hz), 7.11 (1H, s), 7.35 (1H, s), 7.52 - 7.53 (1H, m), 7.62 (2H, d, J 8.8 Hz), 7.71 (1H, s), 8.24 (2H, d, J 8.8 Hz). (Found: C, 56.0; H, 3.1; N, 7.3; S, 8.3; $M^+$, 384.0414 $C_{18}H_{12}N_2O_6S$ requires C, 56.2; H, 3.1; N, 7.3; S, 8.3%; $M^+$, 384.0416).

Example 12(b)

(5RS) Sodium (*E*)-6-(3-Furylmethylene)penem-3-carboxylate

The (*E*)-penem (92) (17 mg) from Example 12(a) was hydrogenated as for Example 1(b) to give, after chromatograpny on Biogel P2 eluting with water and freeze-drying, the title sodium salt (94) (2.8 mg) as an amorphous pale yellow solid, $\lambda_{max.}$ ($H_2O$) 296 nm ($\varepsilon_m$ 16,015).

Example 12(c)

(5RS) Sodium (Z)-6-(3-Furylmethylene)penem-3-carboxylate

The (Z)-penem (93) (70 mg) from Example 12(a) was hydrogenated as for Example 1(b) to give, after chromatography on Biogel P2 eluting with water and freeze-drying, the title sodium salt (95) (25 mg) as an amorphous pale yellow solid, $\lambda_{max.}$ ($H_2O$) 291 nm ($\varepsilon_m$ 17,977); $\nu_{max.}$ (KBr) 3700 - 2500, 1757, 1678, 1596, 1559 $cm^{-1}$; $\delta$ ppm ($D_2O$) 6.55 (1H, d, J 1.8 Hz), 6.68 (1H, d, J 0.9 Hz), 7.09 (1H, s), 7.22 (1H, br.s), 7.63 - 7.65 (1H, m), 7.92 (1H, br. s).

Preparation 13(a)

2-(5-*p*-Nitrobenzyloxycarbonyl-2-furyl)-1,3-dioxolane

A solution of *n*-butyl lithium (1.64 M in *n*-hexane, 6.1 ml) was added to a stirred solution of 2-(2-furyl))-1,3-dioxolane (1.40 g) in dry THF (20 ml) at -76°C under dry argon. After 30 minutes at -76°C the stirred mixture was treated withdry carbon dioxide gas for 3 minutes (the gas was dried by passage through a Drierite drying tower and introduced to the reaction vessel through a wide bore tube close to the surface of the reaction mixture). The cooling bath was removed and the mixture allowed to stand for 30 minutes whilst it attained room temperature. The mixture was diluted with water (50 ml) and washed with ethyl acetate (10 ml). The aqueous layer was acidified with 5% citric acid and extracted with ethyl acetate (3 x 20 ml). The combined extracts were washed with brine (2 x 5 ml), dried ($MgSO_4$), and evaporated to give crude 2-(5-carboxy-2-furyl)-1,3-dioxolane (96) (857 g) as a solid. The crude acid (96) was dissolved in dry dimethylformamide (10 ml) and treated with ahydrous potassium carbonate (321 mg) and *p*-nitrobenzyl bromide (1.01 g). The mixture was stirred at room temperature for 18 hours, diluted with ethyl acetate (60 ml), and washed with brine (3 x 15 ml). The dried ($MgSO_4$) organic layer was evaporated and the residue chromatographed on silica gel eluting with ethyl acetate/hexane mixtures to give, after crystallisation from ethyl acetate/hexane, the title acetal (97) (1.00 g) m.p. 93 - 94°C (rods) $\nu_{max.}$ ($CHCl_3$) 1720 $cm^{-1}$; δ ppm ($CDCl_3$) 4.07 (4H,s), 5.42 (2H, s), 5.98 (2H, d, J 9 Hz), 8.25 (2H, d, J 9 Hz). (Found: C, 56.2; H, 4.2; N, 4.3 $C_{15}H_{13}NO_7$ requires C, 56.4; H, 4.1; N, 4.4%)]

Preparation 13(b)

*p*-Nitrobenzyl 5-Formyl-2-furoate

A mixture of the acetal (97) (319 mg) from Preparation 13(a), pyridine-*p*-toluene sulphonate (25 mg), acetone (5 ml), and water (0.5 ml) were heated under reflux for 24 hours. The mixture was evaporated to low volume, diluted with ethyl acetate (15 ml), and washed with brine (3 x 3 ml). The dried ($MgSO_4$) organic layer was evaporated and the residue crystallised from ethyl acetate/hexane to give the title aldehyde (98) (186 mg) m.p. 100-101°C (plates), $\nu_{max.}$ ($CHCl_3$) 1725, 1690 $cm^{-1}$; $\delta$ ppm ($CDCl_3$) 5.51 (2H, s), 7.25 - 7.45 (2H, m), 7.65 (2H, d, J 9 Hz), 8.29 (2H, d, J 9 Hz), 9.83 (1H, s). (Found: C, 56.8; H, 3.5; N, 5.0. $C_{13}H_9NO_6$ requires C, 56.7; H, 3.3; N, 5.1%).

Preparation 13(c)

1-*t*-Butyldimethylsilyl-3-[hydroxy(5-*p*-nitrobenzyloxycarbonyl-2-furyl)methyl]-4-tritylthioazetidin-2-one

A solution of *n*-butyl lithium (1.6 in *n*-hexane, 0.73 ml) was added to a solution of diisopropylamine (0.17 ml) in dry THF (8 ml) at -30°C under dry argon. After stirring at 30°C for 10 minutes the mixture was cooled to -76°C and treated with a solution of 1-*t*-butyldimethylsilyl-4-tritylthioazetidin-2-one (1) (459 mg) in dry THF (4 ml). After 15 minutes at -76°C the stirred mixture was treated with a solution of the aldehyde (98) (330 mg) from Preparation 13(b) in dry THF (2 ml). After a further 20 minutes at -76°C the stirred mixture was treated with saturated ammonium chloride solution (5 ml) and worked up as for Preparation 1(a) to give two fractions. The less polar fraction contained a *trans*-isomer (Isomer A) of the title azetidinone (99) (180 mg), a solid, m.p. 186-89°C (plates *ex.* ethyl acetate/hexane); $\nu_{max.}$ ($CHCl_3$) 3600 - 3100, 1735 $cm^{-1}$; δ ppm ($CDCl_3$) 0.93 (9H, s), 1.86 (1H, d, J 7 Hz, exch. $D_2O$), 3.44 (1H, dd, J 2 and 2 Hz), 3.61 (1H, dd, J 7 and 2 Hz, collapses to d, J 2 Hz on exch. $D_2O$), 4.49 (1H, d, J 2 Hz), 5.39 (2H, s), 6.49 (1H, d, J 3 Hz), 7.08 - 7.63 (18H, m), 8.23 (2H, d, J 8 Hz), the $Me_2Si$ signals were obscured by the TMS signal. The more polar fraction contained a *trans*-isomer (Isomer B) of the title azetidinone (99) (237 mg), an amorphous solid, $\nu_{max.}$ ($CHCl_3$) 3600 - 3100, 1730 $cm^{-1}$; δ ppm ($CDCl_3$) 0.80 (9H, s), 2.62 (1H, d, J 10 Hz, exch. $D_2O$), 3.72 (1H, br.d, J 6 Hz), 4.03 (1H, br. dd, J 6 and 10 Hz, collapses to d, J 6 Hz on exch. $D_2O$), 4.21 (1H, br.s), 5.35 (2H, br.s), 6.19 (1H, d, J 3 Hz), 7.00 - 7.65 (19H, m), 8.18 (2H, d, J 8 Hz), the $Me_2Si$ signals were obscured by the TMS signal.

Preparation 13(d)

(3RS,4SR) 3-[Acetoxy(5-*p*-nitrobenzyloxycarbonyl-2-furyl)methyl]-1-*t*-butyldimethylsilyl-4-tritylthioazetidin-2-one

The *trans*-azetidinone (99) (Isomer B) (147 mg) from Preparation 13(c) was treated as for Preparation 1(b) to give the title acetate (100) (90 mg) as an amorphous solid, $\nu_{max.}$ ($CHCl_3$) 1740 $cm^{-1}$; $\delta$ ppm ($CDCl_3$) 0.84 (9H, s), 1.94 (3H, s), 3.93 (1H, dd, J 6 and 2 Hz), 4.11 (1H, d, J 2 Hz), 5.28 (1H, d, J 6 Hz), 5.43 (2H, s), 6.32 (1H, d, J 4 Hz), 7.10 - 7.75 (18H, m), 8.28 (2H, d, J 9 Hz), the $Me_2Si$ signals were obscured by the TMS signal.

Preparation 13(e)

(3RS,4SR) 3-[Acetoxy(5-*p*-nitrobenzyloxycarbonyl-2-furyl)methyl]-4-tritylthioazetidin-2-one

The acetate (100) (90 mg) from Preparation 13(d) was treated as for Preparation 4(c) to give the title azetidinone (101) (61 mg), an amorphous solid, $\nu_{max.}$ ($CHCl_3$) 3390, 1775, 1730 sh. $cm^{-1}$; δ ppm ($CDCl_3$) 2.10 (3H, s), 3.81 (1H, slightly broadened dd, J 7 and 3 Hz), 4.40 (1H, br.s), 4.53 (1H, d, J 3 Hz), 5.45 (2H, s), 6.21 (1H, d, J 7 Hz), 6.57 (1H, d, J 4 Hz), 7.20 - 7.70 (18H, m), 8.25 (2H, d, J 9 Hz).

Preparation 13(f)

(3RS,4SR) 3-[Acetoxy(5-p-nitrobenzyloxycarbonyl-2-furyl)methyl]-1-(1-p-nitrobenzyloxycarbonyl-1-triphenylphosphoranylidenemethyl)-4-tritylthioazetidin-2-one

The azetidinone (101) (1.65 g) from Preparation 13(e) was treated as for Preparation 12(e) to give the title phosphorane (102) (2.05 g) as an amorphous solid, $\nu_{max.}$ ($CHCl_3$) 1755, 1620 sh., 1605 $cm^{-1}$.

Preparation 13(g)

(3RS,4SR) Silver 3[Acetoxy(5-*p*-nitrobenzyloxycarbonyl-2-furyl)methyl]-1-(1-*p*-nitrobezyloxycarbonyl-1-triphenylphosphoranylidenemethyl)azetidin-2-one-4-thiolate

The phosphorane (102) (2.05 g) from Preparation 13(f) was dissolved in a mixture of dichloromethane (30 ml) and methanol (10 ml) and treated with silver nitrate/pyridine as for Preparation 1(e) to give the title silver thiolate (103) (1.51 g) as a pale yellow amorphous solid, $\nu_{max.}$ ($CHCl_3$) 1750, 1620 slight shoulder, 1610 $cm^{-1}$.

Preparation 13(h)

(5RS,6SR)p-Nitrobenzyl 6-[Acetoxy(5-p-nitrobenzyloxycarbonyl-2-furyl)methyl]penem-3-carboxylate

The silver thiolate (103) (490 mg) from Preparation 13(g) was treated as for Preparation 1(f) to give the title penem (104) (281 mg) as a solid, m.p. 178-179°C (microcrystalline *ex.* ethyl acetate/hexane); $\nu_{max.}$ ($CHCl_3$) 1795, 1725 $cm^{-1}$; $\delta$ ppm ($CDCl_3$) 2.08 (3H, s), 4.38 (1H, ddd, J 5, 2 and approximatly 1Hz), 5.22 and 5.42 (2H, ABq, J 14 Hz), 5.40 (2H, s), 6.06 (1H, d, J 2 Hz), 6.24 (1H, d, J 5 Hz), 6.59 (1H, d, J 4 Hz), 7.19 (1H, d, J 4 Hz), 7.30 (1H, d, J approximately 1Hz), 7.57 and 7.58 (4H, each d, J 9 Hz), 8.20 and 8.22 (4H, each d, J 9 Hz). (Found: C, 54.0; H, 3.7; N, 6.6; S, 4.9. $C_{28}H_{21}N_3O_{12}S$ requires C, 53.9; H, 3.4; N 6.7; S, 5.1%).

Example 13(a)

(5RS) *p*-Nitrobenzyl (Z)-6-(5-*p*-Nitrobenzyloxycarbonylfurfurylidene)penem-3-carboxylate

The penem (104) (240 mg) from Preparation 13(h) was treated as for Example 1(a) to give the title (Z)-penem (105) (159 mg) as an orange-yellow solid, $\nu_{max.}$ (Nujol) 1770, 1720, 1705, 1680 sh. $cm^{-1}$; δ ppm ($CDCl_3$) 5.30 and 5.46 (2H, ABq, J 13.4 Hz), 5.41 and 5.50 (2H, ABq, J 13.3 Hz), 6.63 (1H, d, J 0.8 Hz), 6.80 (1H, d, J 3.7 Hz), 6.97 (1H, br.s), 7.31 (1H, d, J 3.7 Hz), 7.38 (1H, s), 7.62 (4H, br.d, J approximately 8.2 Hz), 8.25 (d, J 8.4 Hz) and 8.26 (d, J 8.8 Hz), together 4H.

Example 13(b)

(5RS) (Z)-6-(5-Carboxyfurfurylidene)penem-3-carboxylate Disodium Salt

The penem (105) (120 mg) from Example 13(a) was hydrogenated as for Example 1(b) to give, after chromatography on Biogel P2 eluting with water and freeze-drying, the title disodium salt (106) (65 mg) as an orange-yellow amorphous solid, $\lambda_{max.}$ ($H_2O$) 318 nm ($\varepsilon_m$ 19233); $\nu_{max.}$ (Nujol) 3700 - 2000, 1755, 1660 sh., 1600 br., 1560 sh. $cm^{-1}$; $\delta$ ppm ($D_2O$) 6.76 (1H, s), 6.90 (1H, d, J 3.6 Hz), 7.06 (1H, d, J 3.6 Hz), 7.09 (2H, s).

 

Preparation 14(a)

(3RS) 1-(1-allyloxycarbonyl-1-triphenylphosphoranylidenemethyl)-4-tritylthioazetidin-2-one

A solution of 4-tritylthioazetidin-2-one (Bristol-Meyers GB2042515A) (4.35g, 12.6 mmol) in dry DMF (60 ml) was treated with glyoxylic acid hydrate (1.39g, 15.13 mmol). The mixture was stirred with dried 4A molecular sieves for 24h. The reaction mixture was then treated with potassium carbonate (1.04g, 7.56 mmol) and allyl bromide (1.31 ml, 15.13 mmol) and stirred for a further 16 h. The reaction mixture was filtered through kieselguhr, washing the pad well with ethyl acetate. The filtrate was diluted with ethyl acetate, washed well with dilute HCl, and brine then dried ($MgSO_4$) and evaporated. The residue was chromatographed on silica eluting with ethyl acetate/hexane mixtures to give the hydroxy esters (107) (4.8g, 83%) as a white amorphous solid. $\nu$max ($CH_2Cl_2$) 3500, 1770, 1750 $cm^{-1}$.

A stirred solution of the hydroxy esters (107) (4.8g, 10.5 mmol) in dry THF (70 ml) under argon at $-20^{o}$ was sequentially treated with 2,6-lutidine (1.82 ml, 15.7 mmol) and thionyl chloride (1.15 ml, 15.7 mmol). After 15 minutes the mixture was filtered and evaporated. The residue was then dissolved in toluene, treated with charcoal and after 5 minutes filtered and evaporated to give the crude chloroester (108) as a yellow amorphous solid.

The crude chloroester (108) was dissolved in dioxane (100 ml), treated with triphenylphosphine (4.11g, 15.7 mmol) and 2,6-lutidine (1.46 ml, 12.5 mmol) and stirred at $100^{o}$ for 2 h, 16 h at $75^{o}$ and then a further 2 h at $100^{o}$. The mixture was evaporated dissolved in ethyl acetate and washed with dilute HCl, dilute $NaHCO_3$ solution and brine then dried ($MgSO_4$) and evaporated. Chromatography on silica eluting with ethyl acetate/hexane mixtures gave the title phosphorane (109) (3.9g, 53%) as a buff amorphous solid; $\nu$max ($CH_2Cl_2$) 1745, 1610 $cm^{-1}$.

Preparation 14(b)

3-(Hydroxy N-methylpyrrol-2-yl methyl)-1-(1-allyloxycarbonyl-1-triphenylphosphoranylidenemethyl)-4-tritylthioazetidin-2-one

A stirred solution of diisopropylamine (1.12 ml, 8 mmol) in THF (40 ml) under argon at -30$^{o}$ was treated with a solution of n-butyl lithium (1.4M, 5.68 ml, 8 mmol). After 15 minutes the mixture was cooled to -70$^{o}$ and treated dropwise with a solution of the phosphorane (109) (2.8g, 4 mmol) from preparation 14(a) in THF (40 ml). After a further 10 minutes the red mixture was treated with a solution of N-methylpyrrole-2-carboxaldehyde (1.31g, 12 mmol) in THF (4 ml). After a further 10 minutes the reaction mixture was quenched with a saturated aqueous solution of ammonium chloride, and allowed to attain room temperature. The mixture was diluted with ethyl acetate, washed with brine then dried ($MgSO_4$) and evaporated. After chromatography on silica eluting with ethyl acetate/hexane mixtures the later fractions (major low RF spot on t.l.c.) were combined and evaporated to give the title alcohols (110) (470 mg, 14%) as a light yellow amorphous solid; νmax ($CH_2Cl_2$) 3600-3300, 1750, 1610 $cm^{-1}$; $^{1}H$ nmr ($CDCl_3$) shows material contains 2 major isomers ie. *inter alia* δ3.42 (s) and 3.44 (s) (NMe) in about 1:1 ratio.

Preparation 14(c)

Silver 3-(Hydroxy N-methylpyrrol-2-yl methyl)-1-(1-allyloxycarbonyl-1-triphenylphosphoranylidenemethyl)azetidin-2-one-4-thiolate

A stirred solution of the phosphorane (110) (470 mg, 0.58 mmol) from preparation 14(b) in methanol (8 ml) with treated with pyridine (61 µl, 0.75 mmol) and a solution of silver nitrate (0.15M, 5 ml, 0.75 mmol) in MeOH. After 30 minutes the reaction mixture was evaporated to low volume and treated dropwise with ether. The precipitate was collected and dried under vacuum to give the crude title silver thiolate (111) (460 mg, >100%) as a buff solid. This material was pure enough for further synthetic work and was estimated to be 85% pure.

Preparation 14(d)

3-(Hydroxy N-methypyrrol-2-yl methyl)-1-(1-allyloxycarbonyl-1-triphenylphosphoranylidenemethyl)-4-acetylthioazetidin-2-one

A stirred solution of the crude silver thiolate (111) (360 mg, 85% pure, 0.625 mmol) in dry acetonitrile (36 ml) was treated with a solution of acetyl chloride (60 μl, 0.812 mmol) in acetonitrile (0.5 ml). Within 1 minute precipitation occurred. After a further 2 minutes the mixture was filtered through Kieselguhr, washing the pad well with ethyl acetate. The filtrate was diluted with ethyl acetate and washed with dilute $NaHCO_3$ solution and brine. The dried ($MgSO_4$) organic phase was evaporated and chromatographed on silica eluting ethyl acetate/hexane mixtures and then ethyl acetate. The later fractions, containing a polar material, were combined and evaporated to give the title thioester (112) (50 mg, 13%) as a light yellow amorphous solid; νmax ($CH_2Cl_2$) 3600-3200, 1750, 1695, 1620 $cm^{-1}$.

Preparation 14(e)

Allyl 6-(Hydroxy N-methylpyrrol-2-yl methyl)-2-methylpenem-3-carboxylate

A stirred solution of the thioester (112) (50 mg) from preparation 14(d) in toluene (50 ml) was heated at 110° under argon for 1 hour. The cooled reaction mixture was evaporated to volume and chromatographed on silica eluting with 40% ethyl acetate/hexane to give the title penem (113) (6.4 mg) as a light yellow amorphous solid; νmax ($CH_2Cl_2$) 3600-3300, 1785, 1710, 1590 $cm^{-1}$. $^1H$ Nmr showed that the material was essentially a single trans isomer - δ($CDCl_3$) 2.05 (1H, d, *J* 6.7Hz) 2.38 (3H, s), 3.69 (3H, s), 4.24 (1H, dd, *J* 1.7, 7.1Hz), 4.66 (1H, dd, *J* 5.6, 13.4Hz), 4.79 (1H, dd, *J* 5.4, 13.4Hz), 5.10 (1H, t, *J* 6.9Hz), 5.26 (1H, dd, *J* 1.4, 10.4Hz), 5.39 (1H, dd, *J* 1.4, 17.2Hz), 5.43 (1H, d, *J* 1.6Hz), 5.96 (1H, ddd, *J* 5.5, 10.4, 17.2Hz), 6.08 (1H, dd, *J* 2.8, 3.5Hz), 6.24 (1H, dd, *J* 1.5, 3.5Hz), 6.64-6.66 (1H, m).

The presence of a cis isomer (~5%) was indicated by *inter alia* δ 2.35 (s), 3.74 (s), 4.36 (dd, *J* 6.4, 16.0Hz), 5.66 (d, *J* 6.4 Hz).

Example 14

(5RS) Allyl 2-methyl-6-N-methylpyrrol-2-ylmethylenepenem-3-carboxylate

A stirred solution of the penem (113) (8.4 mg, 0.025 mmol) from preparation 14(e) in dry dichloromethane (1 ml) at $0^o$ under argon was sequentially treated with a solution of triethylamine (7 µl, 0.050 mmol) in dichloromethane (0.1 ml) and a solution of methanesulphonyl chloride (3.9 µl, 0.050 mmol) in dichloromethane (0.1 ml). After 30 minutes the mixture was treated with further qunatities of triethylamine (3.5 µl, 0.025 mmol) in dichloromethane (0.05 ml) and methanesulphonyl chloride (1.95 µl, 0.025 mmol) in dichloromethane (0.05 ml). After a further 30 minutes the mixture was again treated a solution of triethylamine (3.5 µl, 0.025 mmol) in dichloromethane (0.05 ml) and methanesulphonyl chloride (1.95 µl, 0.025 mmol) in dichloromethane (0.05 ml). After a further 30 minutes the yellow reaction mixture was diluted with dichloromethane, washed with 5% citric acid solution, dilute $NaHCO_3$ solution and brine then dried ($MgSO_4$) and evaporated. The residue was chromatographed on silica eluting with 30% ethyl acetate/hexane to give the title penem (114) (5 mg) as a 6:1 mixture of E : Z isomers; νmax ($CH_2Cl_2$) 1765, 1710, 1650, 1590 $cm^{-1}$; δ($CDCl_3$) (an approx. $6^o$:$1^+$ ratio of $E^o$:$Z^+$ isomers) $2.36^o$ (s) and $2.37^+$ (s) (together 3H), $3.68^o$ (s) and $3.70^+$ (s) (together 3H), 4.70 (1H, dd, *J*5.6, 13.4Hz), 4.82 (1H, dd, *J* 5.4, 13.4Hz), 5.27 (1H, dd, *J* 1.4, 10.4Hz), 5.45 (1H, dd, *J* 1.4, 17.2 Hz), 6.00 (1H, ddd, *J* 5.5, 10.4, 17.2Hz), 6.22 (s) overlaying 6.18-6.27 (m) (together 2H), $6.46^o$ (6/7H, s), 6.75-$6.77^o$ (6/7H, m), 6.83-$6.85^+$ (1/7H, m) $7.06^+$ (1/7H, s), 7.60 (1H, dd, *J* 1.5, 4.1Hz).

Preparation 15(a)

t-Butyldiphenylsilyloxyacetyl chloride

Oxalyl chloride (2.27 ml) was added to a solution of O-t-butyldiphenylsilylglycollic acid (Beecham, GB 2037277B) (6.28g) and dimethylformamide (3 drops) in dry dichloromethane (30 ml) at $0^{o}C$. The mixture was kept at room temperature for 1 hour and treated with a further portion (3 drops) of dimethylformamide. The mixture was stirred until the reaction was complete (infra-red monitoring). The mixture was evaporated to give the title acid chloride (115) (6.2g) as an oil, $\nu$max ($CH_2Cl_2$) 1810 $cm^{-1}$; $\delta$ppm ($CDCl_3$) 1.10 (9H,s), 4.50 (2H, s), 7.0-8.0 (10H, m).

Preparation 15(b)

(3RS, 4SR) 3-[Acetoxy (3-furyl)methyl]-4-t-butyldiphenylsilyloxyacetylthio-1-(1-p-nitrobenzyloxycarbonyl-1-triphenylphosphoranylidenemethyl) azetidin-2-one

The acid chloride (115) (250 mg) from Preparation 15(a) was added to a stirred, ice bath cooled, mixture of the silver salt (90) (200 mg) from Preparation 12(g) and pyridine (59 mg) in dry dichloromethane (5 ml). After stirring at ice bath temperature for 10 minutes the mixture was filtered through Kieselguhr and the residue was washed with ethyl acetate (2 x 10 ml). The combined filtrate were washed with 1N. hydrochloric acid (2 ml), brine (2 ml), saturated $NaHCO_3$ solution (2 ml) and brine (3 x 2 ml). The dried ($MgSO_4$) organic layer was evaporated and the residue chromatographed on silica gel eluting with ethyl acetate/hexane mixtures to give the title phosphorane (116) (189 mg) as an amorphous solid, $\nu max$ ($CHCl_3$) 1760, 1690, 1620, 1605 sh. $cm^{-1}$.

Preparation 15(c)

(5RS, 6SR) p-Nitrobenzyl 6-[Acetoxy(3-furyl)methyl]-2-t-butyldiphenylsilyloxymethylpenem-3-carboxylate

The phosphorane (116) (189 mg) from Preparation 15(b) was heated in refluxing toluene (40 ml) under argon for 2 hours. The mixture was evaporated and the residue was chromatographed on silica gel eluting with ethyl acetate/hexane mixtures to give the title penem (117) (106 mg) as an amorphous solid, λmax ($EtOH/CHCl_3$) 259 (Em 12572), 263 (12610) and 319 nm (8727); νmax ($CHCl_3$) 1790, 1740, 1705 $cm^{-1}$; δppm ($CDCl_3$) 1.05 (9H, s), 2.11 (3H, s), 4.23 (1H, dd, J6.2 and 1.8Hz), 4.84 (2H, s), 5.11 and 5.27 (2H, ABq, J13.8Hz), 5.59 (1H, d, J1.8Hz), 6.21 (1H, d, J6.2Hz), 6.42 (1H, d, J1.1Hz), 7.3-7.7 (14H, m), 8.13 (2H, d, J8.7Hz).

Preparation 15(d)

(5RS, 6SR) p-Nitrobenzyl 6-[Acetoxy(3-furyl)methyl]-2-hydroxymethyl-penem-3-carboxylate

The penem (117) (300 mg) from Preparation 15(c) was dissolved in dry THF (10 ml), cooled in an ice bath, and treated with glacial acetic acid (76 mg) and tetra-n-butylammonium fluoride (0.55 ml of a 1M. solution in THF). After 30 minutes at ice bath temperature the mixture was diluted with ethyl acetate (40 ml) and washed with brine (3 ml), saturated $NaHCO_3$ solution (3 ml) and brine (3 x 3 ml). The dried ($MgSO_4$) organic layer was evaporated and the residue chromatographed on silica gel eluting with ethyl acetate/hexane mixtures to give the title penem (118) (182 mg) as a solid, mp. 138 - 140$^oC$ (rhomboids ex ethyl acetate/hexane); λmax (EtOH/$CHCl_3$) 262 (Em 12223) and 319 nm (9312); νmax ($CHCl_3$) 3600-3100, 1795, 1740, 1705 $cm^{-1}$; δ ppm ($CDCl_3$) 2.10 (3H, s), 3.23 (1H, t, J6.8Hz), 4.25 (1H, dd, J6.0 and 1.7Hz), 4.63-4.66 (2H, m), 5.26 and 5.43 (2H, ABq, J13.8Hz), 5.61 (1H, d, J1.7Hz), 6.21 (1H, d, J6.0Hz), 6.40 br(1H, s), 7.43 (1H, dd, J1.5 and 1.5Hz), 7.50 br(1H, s), 7.61 (2H, d, J8.6Hz), 8.24 (2H, d, J8.6Hz).

Example 15(a)

(5RS) p-Nitrobenzyl 6-(3-Furylmethylene)-2-hydroxymethylpenem-3-carboxylate

A solution of DBU (80 mg) in dry dichloromethane (1 ml) was added, dropwise over 1 minute, to a stirred solution of the penem (118) (100 mg) from Preparation 15(d) in dry dichloromethane (4 ml) at -40°C. After stirring at -40°C for 30 minutes the mixture was diluted with dichloromethane (5 ml) and washed with 5% citric acid (1 ml), brine (1 ml), saturated $NaHCO_3$ solution (1 ml) and brine (3 x 1 ml). The dried ($MgSO_4$) organic layer was evaporated to give a mixture of the (E) and (Z)-isomers of the title penem (119). Careful chromatography of the mixture on silica gel eluting with dichloromethane/ethyl acetate mixtures allowed the separation of the (Z)-isomer of the title penem (119) (63 mg), a solid, mp. 168-169°C (fine needles ex chloroform/hexane); λmax ($EtOH/CHCl_3$) 293 nm (Em 25,697); νmax ($CHCl_3$) 3500-2500, 1790, 1685, 1670 $cm^{-1}$; δppm ($CDCl_3$) 3.15 (1H, t, J6.9Hz), 4.62-4.68 (2H, m), 5.28 and 5.50 (2H, ABq, J13.7Hz), 6.33 br(1H, s), 6.37 (1H, d, J0.9Hz), 7.10 (1H, s), 7.52 (1H, dd, J1.7 and 1.7Hz), 7.67 (2H, d, J8.8Hz), 7.71 (1H, s), 8.25 (2H, d, J8.8Hz). (Found: C, 54.8; H, 3.5; N, 6.5; S, 7.7. $C_{19}H_{14}N_2O_7S$ requires C, 55.1; H, 3.4; N, 6.8; S, 7.7%.

Example 15(b)

(5RS) Sodium (Z)-6-(3-Furylmethylene)-2-hydroxymethylpenem-3-carboxylate

The (Z)-penem ester (119) (48 mg) from Example 15(a) was hydrogenated as for Example 1(b) to give, after chromatography on Biogel P2 eluting with water and freeze drying, the title sodium salt (120) (21 mg) as a pale yellow amorphous solid, λmax ($H_2O$) 288 nm (Em 15127), νmax (KBr) 3650-2600, 1755, 1680, 1600, 1570 $cm^{-1}$; δppm ($D_2O$) 4.54 and 4.76 (2H, ABq, J14.9Hz), 6.51 (1H, s), 6.55 br (1H, s), 7.21 (1H, s), 7.63 br(1H,s), 7.91 br(1H, s).

The following tables illustrate the activity of the compounds.

Although many broad spectrum cephalosporins are generally active against many organisms which are traditionally regarded as resistant, certain strains have been emerging recently which are resistant to these newer cephalosporins also. Table 3 illustrates how the present compounds render these strains sensitive.

Table 1

The Effect of Alkylidene-penems on the MIC of Amoxycillin and Cephaloridine Against β-Lactamase-Producing Organisms

| | Conc + of Inhib | MIC (μg/ml) of *antibiotic against - | | | |
|---|---|---|---|---|---|
| | | S Aureus Russell | Kleb aerogenes | E.coli RTEM | E.coli JT410 |
| tibiotic Alone | - | 500 | 500 | 2000 | 62-125 |
| tibiotic + Compound of Example 1b | 1.0<br>5.0 | 0.31<br>- | -<br>3.1 | -<br>1.0 | -<br>4.0 |
| tibiotic + Compound of Example 2b | 1.0<br>5.0 | 0.25<br>- | -<br>40 | -<br>12.5 | -<br>6.2 |
| tibiotic + Compound of Example 3b | 1.0<br>5.0 | 0.16<br>- | -<br>50 | -<br>16 | -<br>8.0 |
| tibiotic + Compound of Example 5b | 1.0<br>5.0 | 0.16<br>- | -<br>100 | -<br>12.5 | -<br>16 |
| tibiotic + Compound of Example 7b | 10<br>5.0 | 0.6<br>- | -<br>25 | -<br>4.0 | -<br>8.0 |
| tibiotic + Compound of Example 8b | 1.0<br>5.0 | 0.3<br>- | -<br>3.1 | -<br>2.0 | -<br>8.0 |
| tibiotic + Compound of Example 9b | 1.0<br>5.0 | 2.5<br>- | -<br>3.1 | -<br>8.0 | -<br>16 |
| tibiotic + Compound of Example 11b | 1.0<br>5.0 | 1.25<br>- | -<br>12.5 | -<br>8.0 | -<br>31 |
| tibiotic + Compound of Example 12b | 1.0<br>5.0 | 0.6<br>- | -<br>12.5 | -<br>2.0 | -<br>8.0 |

ncentration of inhibitor (in μg/ml) added to antibiotic.
oxycillin used against S. aureus Russell, Klebsiella aerogenes and E. coli JT39 ($R_{TEM}$)
phaloridine used against E. coli JT410
ne inhibitors had no activity alone at the concentrations tested (i.e. 5 and 1 μg/ml)

Table 2

Antibacterial Activity of Alkylidene-penems

Against Staphylococci

MIC (μg/ml)

| Compound of Example No | Russell | Oxford |
| --- | --- | --- |
| 1b | 16.0 | 16.0 |
| 2b | 10.0 | 5.0 |
| 3b | 12.5 | 12.5 |
| 5b | 12.5 | 3.1 |
| 7b | 50 | 100 |
| 8b | 12.5 | 50 |
| 9b | 125 | 62 |
| 11b | 50 | 50 |
| 12b | 50 | 100 |

Table 3

Activity of Compound of Example 1b with Broad Spectrum Cephalosporins against a range of resistant strains

(MIC Cephalosporin µg/ml)

| Organism | Strain | Cefotaxime alone | + Ex 1b 20 µg/ml | + Ex 1b 5 µg/ml | Ceftazidine alone | + Ex 1b 20 µg/ml | + Ex 1b 5 µg/ml |
|---|---|---|---|---|---|---|---|
| Ps.aeruginosa | Charlier | >80 | >20 | >20 | 80 | >20 | >20 |
| | Badia IR | >80 | >20 | >20 | >80 | 5 | 20 |
| S.marcescens | V697 | >80 | 0.6 | 2.5 | 80 | 1.25 | 2.5 |
| | HCN 3956 | 80 | 2.5 | 2.5 | 5 | 2.5 | 5 |
| C.freundii | T1739 | 40 | 0.08 | 0.15 | 80 | 0.15 | 0.3 |
| | 423 | 40 | 0.15 | 0.15 | 80 | 0.3 | 0.3 |
| | T562 | 40 | 0.08 | 0.3 | 80 | 0.3 | 0.6 |
| M.morganii | 978 | >80 | 0.08 | 0.08 | 2.5 | 0.15 | 0.3 |
| E.cloacae | 4508 | >80 | 0.6 | 2.5 | >80 | 0.6 | 2.5 |
| | V692/80 | >80 | 0.6 | 2.5 | >80 | 1.25 | 2.5 |
| | P383 | >80 | 0.6 | 2.5 | >80 | 0.6 | 1.25 |
| | 159/167 | >80 | 1.25 | 5 | >80 | 1.25 | 2.5 |
| | Dokt 5 | >80 | 0.6 | 2.5 | >80 | 0.6 | 1.25 |
| E.aerogenes | 53 | >80 | 0.15 | 0.3 | >80 | 0.6 | 1.25 |
| | 55 | 80 | 0.3 | 0.3 | 80 | 0.6 | 1.25 |
| | P99 | >80 | 0.08 | 0.3 | 80 | 0.3 | 0.6 |

Microtitre technique - dilution in TSB
Inoc = approx 2E6 EFU/ml (= approx 2E5 CFU/well)
MIC Ex 1b alone = >80µg/ml

Claims

1) A compound of formula (II):

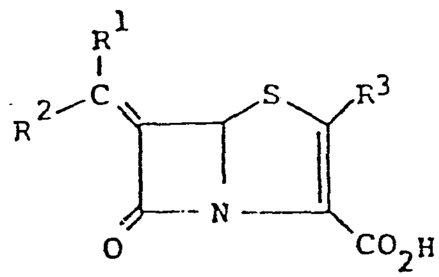

(II)

or a pharmaceutically acceptable salt or in vivo hydrolysable ester thereof, wherein one of $R^1$ or $R^2$ is hydrogen, and the other is a group of subformula (a):

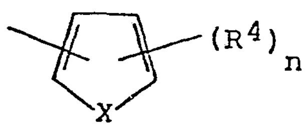

(a)

wherein $R^4$ is a substituent group, X is an oxygen atom, a sulphur atom or an $-NR^5$ group wherein $R^5$ is hydrogen hydrocarbon or a nitrogen protecting group, and n is 0, 1, 2 or 3;
and $R^3$ represents hydrogen or an organic group.

2) A compound according to claim 1 wherein one of $R^1$ or $R^2$ is a group of sub-formula (a) in which n is zero.

3) A compound according to claim 1 or claim 2 wherein one of $R^1$ or $R^2$ is 2-furyl, 3-furyl, 2-thienyl or 3-thienyl.

4) A compound according to claim 3 wherein one of $R^1$ or $R^2$ is 2-furyl.

5) A compound according to any one of the preceding claims wherein $R^3$ is hydrogen or a group of formula $R^a$ or $SR^a$ wherein $R^a$ is an optionally substituted $C_{1-10}$ hydrocarbon or heterocyclic group.

6) A process for preparing a compound of formula (II) as defined in claim 1 or a pharmaceutically acceptable salt or in vivo hydrolysable ester thereof which process comprises the elimination of the elements H-Z from a compound of formula (III)

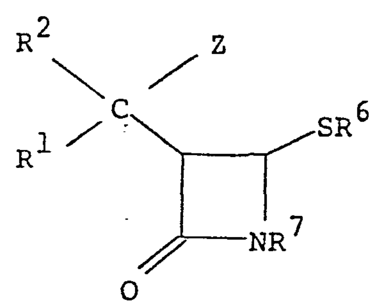

(III)

wherein $R_1$ and $R^2$ are as defined as claim 1;

Z is hydroxy or a leaving group;

$R^6$ is $C_{1-6}$ alkyl, aryl or aryl ($C_{1-6}$) alkyl;

$R^7$ is hydrogen or an N-protecting group; or $R^6$ and $R^7$ are joined together to form a penem ring such that the compound is of formula (IV)

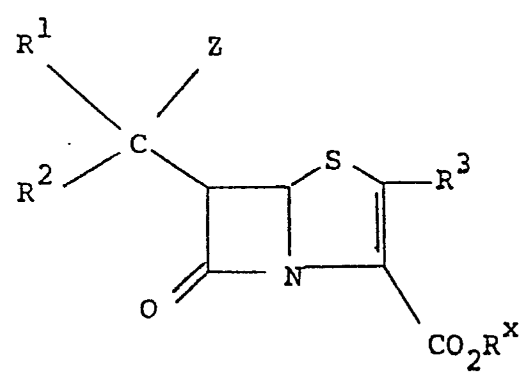

(IV)

wherein $R^1$, $R^2$ and $R^3$ are as defined in claim 1, Z is as defined above; and

$R^1$ is a carboxy blocking group; and thereafter if necessary carrying out one or more of the following steps:

(i) joining $R^6$ and $R^7$ to complete the penem ring of formula (II);

(ii) removing the carboxy blocking group $R^x$; and

(iii) converting the product into a pharmaceutically acceptable salt or in vivo hydrolysable ester.

7) A process for preparing a compound of formula (II) wherein $R^3$ is a group $SR^a$, which process comprises reacting a sulphoxide of formula (X)

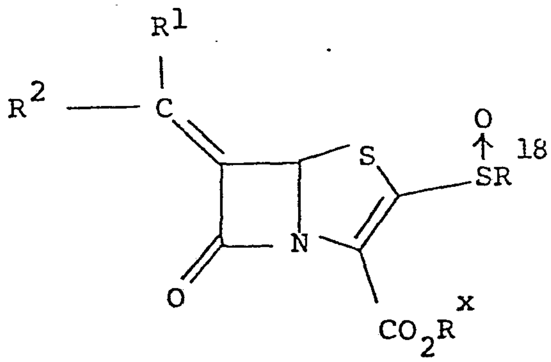

(X)

wherein $R^1$ and $R^2$ are as defined in claim 1 and $R^x$ is as defined in claim 6 and $R^{18}$ is an organic group different to the group $R^a$; with a thiol of formula (XI)

$$R^a - SH \qquad (XI)$$

or a reactive derivative thereof and thereafter carrying out one or more of the following steps:

i) removal of any carboxyl blocking group $R^x$;

ii) converting the product into a pharmaceutically acceptable salt or in-vivo hydrolysable ester group.

8) A pharmaceutical composition comprising a compound of formula (II) as defined in claim 1 or a pharmaceutically acceptable salt or in vivo hydrolysable ester thereof, and a pharmaceutically acceptable carrier.

9) A pharmaceutical compoisition according to claim 8 and which further comprises a penicillin or cephalosporin.

10) A compound of formula (II) as defined in claim 1 or a pharmaceutially acceptable salt or ester thereof for use in the treatment of bacterial infection.

11) A compound of formula (IXa):

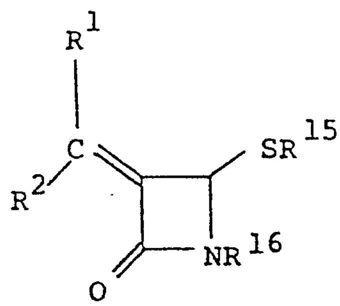

(IXa)

wherein $R^1$ and $R^2$ are as defined in claim 1, $R^{15}$ is $C_{1-6}$ alkyl, aryl or aryl $(C_{1-6})$ alkyl and $R^{16}$ is hydrogen or an N-protecting group.

European Patent Office

**0120613**

Application number

# EUROPEAN SEARCH REPORT

EP 84 30 1255

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 050 805 (HOFFMANN-LA ROCHE) * Claims * | 1,8-10 | C 07 D 499/00<br>C 07 D 405/06<br>C 07 D 409/06<br>A 61 K 31/43 //<br>C 07 D 407/04<br>C 07 D 307/68<br>C 07 F 7/10<br>C 07 F 7/18<br>C 07 F 9/65 |
| A | SYNTHESIS, INTERNATIONAL JOURNAL OF METHODS IN SYNTHETIC ORGANIC CHEMISTRY, no. 11, November 1980, pages 933-935, Georg Thieme Verlag, Stuttgart, DE; T. AGAWA et al.: "Novel synthesis of alpha-methylene-beta-lactams" * Page 933, column 2 * | 11 | |
| D,A | EP-A-0 041 768 (BEECHAM) * Claims * | 1-10 | |

TECHNICAL FIELDS SEARCHED (Int. Cl. 3)

C 07 D 499/00
C 07 D 405/00
C 07 D 409/00
C 07 D 403/00
C 07 D 205/00
A 61 K 31/00

The present search report has been drawn up for all claims

| Place of search | Date of completion of the search | Examiner |
|---|---|---|
| THE HAGUE | 22-06-1984 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document